# EUROPEAN PATENT APPLICATION

(11) **EP 4 109 471 A1**
(43) Date of publication of application: **28.12.2022**
(21) Application number: 22164230.9
(22) Date of filing: 24.03.2022
(51) Int. Cl.: G16H 80/00, G16H 40/67

(54) **REMOTE CARE MANAGEMENT**

(30) Priority: 26.06.2021 US 202163166382 P
(71) Applicant: Hill-Rom Services, Inc., Batesville, IN 47006-9167 (US)
(72) Inventor: CHAHAL, Jotpreet, Batesville, 47006-9167 (US); CRESS, Cameron, Batesville, 47006-9167 (US); GUNN, Jennifer Ann, Batesville, 47006-9167 (US); HOWELL, Benjamin E, Batesville, 47006-9167 (US); JIVA, Gabriel, Batesville, 47006-9167 (US); JULIAN, Jonathan, Batesville, 47006-9167 (US); KEEGAN, Christopher M, Batesville, 47006-9167 (US); MEYERSON, Craig, Batesville, 47006-9167 (US); SCHWENCER, Karrie, Batesville, 47006-9167 (US); SHIRLEY, Daniel, Batesville, 47006-9167 (US)
(74) Representative: Findlay, Alice Rosemary

(57) **Abstract**

A device for remote care management provides a screen configurable by a caregiver to create a virtual care request. The device receives a selection on the screen to submit the virtual care request. The device receives an acceptance of the virtual care request from a remote care provider. The device provides a connection for the caregiver to enter a virtual patient room with the remote care provider.

## Description

Certain clinical care environments such as rural community hospitals can have difficulty in hiring and training medical specialists such as intensivists, neurologists, cardiologists, psychologists, and the like due to cost and geographical constraints. This can inhibit these clinical care environments from providing urgent care to patients.

In some scenarios, a shortage of trained medical specialists can make it necessary to transfer a patient in need of acute care to another medical facility that staffs a desired medical specialist for treating the patient. Not only is this costly and inconvenient for the patient, but it can also delay clinical intervention and thereby lead to patient deterioration.

In general terms, the present disclosure relates to remote care management. In one possible configuration, a device operated by a caregiver submits a virtual care request that is received by a device operated by a remote care provider, and the remote care provider can accept the virtual care request and enter a virtual patient room with the caregiver. Various aspects are described in this disclosure, which include, but are not limited to, the following aspects.

In one aspect, a device for remote care management comprises: at least one processor; and a memory storing instructions which, when executed by the at least one processor, cause the device to: provide a screen configurable by a caregiver to create a virtual care request; receive a selection on the screen to submit the virtual care request; receive an acceptance of the virtual care request from a remote care provider; and provide a connection for the caregiver to enter a virtual patient room with the remote care provider.

In another aspect, a method of remote care management comprises providing a screen configurable by a caregiver to create a virtual care request; receiving a selection to submit the virtual care request; receiving an acceptance of the virtual care request from a remote care provider; and providing a connection for the caregiver to enter a virtual patient room with the remote care provider.

In another aspect, a device for remote care management comprises: at least one processor; and a memory storing instructions which, when executed by the at least one processor, cause the device to: receive a virtual care request; display information added by a caregiver to the virtual care request; display controls to decline or accept the virtual care request; and in response to receiving an acceptance of the virtual care request, provide a connection for a remote care provider to enter a virtual patient room with the caregiver.

In another aspect, a method of remote care management comprises receiving a virtual care request; displaying information added by a caregiver to the virtual care request; displaying controls to decline or accept the virtual care request; and providing a connection for a remote care provider to enter a virtual patient room with the caregiver when the remote care provider accepts the virtual care request.

The invention will now be further described by way of example with reference to the accompanying drawings, in which:
FIG. 1 is a schematic diagram of a system that includes devices each operating a virtual care management application for managing consultations between a caregiver and remote care providers.
FIG. 2 illustrates an example of a method of requesting a consultation with a remote care provider using the virtual care management application installed on a device of the caregiver.
FIG. 3 illustrates an example of a sign in screen generated on the primary device of the caregiver by the virtual care management application.
FIG. 4 illustrates an example of a sign in screen that is generated on the device of the caregiver by the virtual care management application in response to the caregiver selecting a sign in icon of the sign in screen shown in FIG. 3.
FIG. 5 illustrates an example of a sign in screen generated on the device of the caregiver by the virtual care management application in response to the caregiver selecting a first option of the sign in screen of FIG. 4.
FIG. 6 illustrates an example of a sign in screen generated on the device of the caregiver by the virtual care management application in response to the caregiver selecting a second option of the sign in screen of FIG. 4, or by selecting a scan badge icon of the sign in screen of FIG. 5.
FIG. 7 illustrates an example of a care requests screen that is generated on the device of the caregiver upon successfully signing into the virtual care management application.
FIG. 8 illustrates in more detail an operation of submitting a virtual care request that is part of the method of FIG. 2.
FIG. 9 illustrates an example of a create new care request screen that is generated on the device of the caregiver by the virtual care management application in response to the caregiver selecting the create new request icon of the care requests screen of FIG. 7.
FIG. 10 illustrates an example of a patient identification screen generated on the device of the caregiver by the virtual care management application in response to the caregiver selecting a link from the create new care request screen of FIG. 9.
FIG. 11 illustrates another example of a create new care request screen that can be used by the caregiver to identify a patient.
FIG. 12 illustrates an example of a create new care request screen generated on the device of the caregiver by the virtual care management application after a patient is identified.
FIG. 13 illustrates an example of a create new care request screen that is generated on the device of the caregiver by the virtual care management application.
FIG. 14 illustrates an example of a create new care request screen that is generated on the device of the caregiver by the virtual care management application.
FIG. 15 illustrates an example of a care requests screen that is generated on the device of the caregiver by the virtual care management application after the caregiver selects a submit icon from the create new care request screen of FIG. 14.
FIG. 16 illustrates an example of a care request screen generated on the device of the caregiver by the virtual care management application in response to the caregiver selecting a care request from the care requests screen of FIG. 15.
FIG. 17 an example of a care request screen that includes an edit tab generated in response to the caregiver selecting an edit icon on the care request screen of FIG. 16.
FIG. 18 illustrates an example of a care requests screen generated on the device of the caregiver by the virtual care management application.
FIG. 19 illustrates an example of a notification generated on the device of the caregiver by the virtual care management application.
FIG. 20 illustrates an example of a care request screen generated on the device of the caregiver by the virtual care management application.
FIG. 21 illustrates an example of a connection screen generated on a primary device of the caregiver by the virtual care management application in response to the caregiver selecting a join meeting room icon from the care request screen of FIG. 16.
FIG. 22 illustrates an example of a meeting room screen generated on the primary device of the caregiver by the virtual care management application in response to the caregiver selecting a first connection option from the connection screen of FIG. 21.
FIG. 23 illustrates an example of a connection transfer screen generated on the primary device of the caregiver by the virtual care management application in response to the caregiver selecting a second connection option from the connection screen of FIG. 21.
FIG. 24 illustrates an example of a connection transfer screen generated on a secondary device of the caregiver by the virtual care management application.
FIG. 25 illustrates an example of a meeting room screen generated on the secondary device of the caregiver by the virtual care management application after a virtual patient room has been successfully transferred from the primary device to the secondary device.
FIG. 26 illustrates another example of a meeting room screen generated on the secondary device of the caregiver by the virtual care management application after a remote care provider has joined a virtual patient room.
FIG. 27 illustrates an example of a method of providing a consultation to a caregiver using the virtual care management application installed on a device of the remote care provider.
FIG. 28 illustrates an example of a home screen generated on a primary device of the remote care provider after the remote care provider has successfully signed into the virtual care management application.
FIG. 29 illustrates an example of a care requests screen generated on the primary device of the remote care provider in response to the remote care provider selecting a view care requests icon on the home screen of FIG. 28.
FIG. 30 illustrates an example of a patient details screen generated on the primary device of the remote care provider in response to selecting a view icon in a new care request displayed on the care requests screen of FIG. 29.
FIG. 31 illustrates an example of a patient details screen generated on the primary device of the remote care provider in response to the remote care provider selecting an accept icon to accept the care request of the patient details screen shown in FIG. 30.
FIG. 32 illustrates another example of a patient details screen generated on the primary device of the remote care provider.
FIG. 33 illustrates an example of a patient details screen generated on the primary device of the remote care provider in response to the remote care provider selecting a join meeting room icon from the patient details screen of FIG. 31.
FIG. 34 illustrates an example of a notification displayed on a secondary device of the remote care provider by the virtual care management application.
FIG. 35 illustrates an example of a sign in screen generated on the secondary device of the remote care provider by the virtual care management application.
FIG. 36 illustrates an example of a care requests screen generated on the secondary device of the remote care provider.
FIG. 37 illustrates an example of an accepted care request screen generated on the secondary device of the remote care provider in response to the remote care provider selecting an accept icon on the new care request of FIG. 36.
FIG. 38 illustrates an example of a meeting room screen generated on the secondary device of the remote care provider in response to the remote care provider selecting a join meeting room icon from the meeting room module of FIG. 37.
FIG. 39 illustrates an example of an all care requests screen generated on the primary device of the remote care provider in response to the remote care provider selecting an all care requests tab included in the care requests screen of FIG. 29.
FIG. 40 schematically illustrates an example of a device from the system of FIG. 1 that can be used by a caregiver or remote care provider to implement aspects of the virtual care management application.

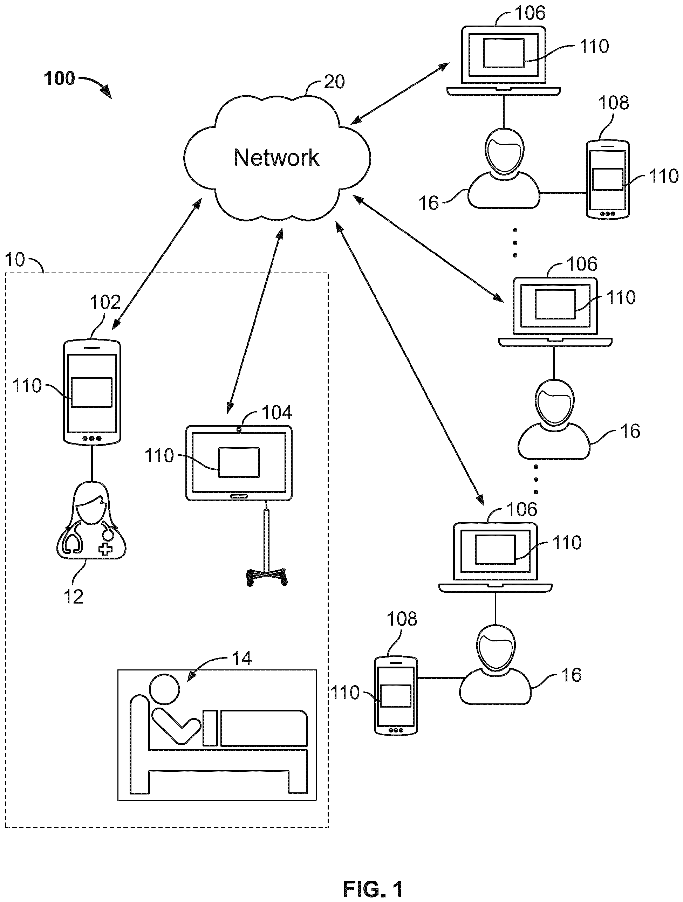

FIG. 1 is a schematic diagram of a system 100 that includes devices 102, 104, 106, 108 that each operate a virtual care management application 110 for managing consultations between a caregiver 12 and remote care providers 16. As shown in FIG. 1, the caregiver 12 provides care to a patient 14 inside a clinical care environment 10. In some examples, the caregiver 12 is consider a local caregiver of the clinical care environment 10. In some further examples, the clinical care environment 10 is located in a rural, sparsely populated area.

As shown in FIG. 1, the remote care providers 16 are located outside of the clinical care environment 10, and are remotely located with respect to the caregiver 12 and patient 14. As an illustrative example, the remote care providers can be located in a different city, county, or state from the location of the clinical care environment 10. Also, the remote care providers 16 can be located remotely with respect to one another. For example, remote care provider 16a can be located in a different city, county, or state than remote care providers 16b, 16c.

In some examples, the remote care providers 16 are medical specialists such as an intensivist, a neurologist, a cardiologist, a psychologist, and the like. In some further examples, a remote care provider 16 is an interpreter/translator, or other kind of provider.

In certain examples, the virtual care management application 110 is installed on the devices 102, 104, 106, 108. Alternatively, the virtual care management application 110 can be a web-based or cloud-based application that is accessible on the devices 102, 104, 106, 108.

The virtual care management application 110 enables the caregiver 12 to provide acute care for the patient 14 by allowing the caregiver 12 to connect and consult with a remote care provider 16 who is not physically located in the clinical care environment 10. Advantages for the patient 14 can include reducing the need to transfer the patient 14 to another clinical care environment or location, and minimizing patient deterioration through faster clinical intervention. Advantages for the caregiver 12 can include receiving mentorship and assistance with documentation and cosigning of medication administration. Advantages for the remote care provider 16 can include allowing the remote care provider 16 to cover more patients over a wider geographical area while working from a single, convenient location.

As shown in FIG. 1, the caregiver 12 can use both a primary device 102 and a secondary device 104 that each have the virtual care management application 110 installed thereon, or otherwise are able to access the virtual care management application 110 when hosted online or in a cloud computing network. In the example illustrated in the figures, the primary device 102 is a mobile device such as a smartphone that the caregiver 12 carries with them as they perform rounding and provide patient care in the clinical care environment 10.

The secondary device 104 can be a workstation such as a tablet computer, or a display monitor attached to a mobile stand that can be carted around the clinical care environment 10. The secondary device 104 can be shared with other caregivers in the clinical care environment 10. In some examples, the secondary device 104 can be a smart TV located in the patient's room, which is configured to access the virtual care management application 110.

The primary and secondary devices 102, 104 are interchangeable with one another. For example, in some alternative examples the secondary device 104 can be a smartphone carried by the caregiver 12, and the primary device 102 can be a workstation such as a tablet computer, a display monitor attached to a mobile stand, or a smart TV.

The remote care providers 16 can similarly use both a primary device 106 and a secondary device 108 that can each access the virtual care management application 110. In the example illustrated in the figures, the primary device 106 of the remote care provider 16 is a laptop, a tablet computer, or a desktop computer, and the secondary device 108 is a smartphone. The primary and secondary devices 106, 108 are interchangeable such that in some examples the secondary device 104 can be a laptop, a tablet computer, or a desktop computer, and the primary device 102 is a smartphone that the remote care provider carries with them.

The consultations between the caregiver 12 and the remote care providers 16 are managed across a communications network 20. As shown in the example of FIG. 1, the primary and secondary devices 102, 104 used by the caregiver 12 are connected to the communications network 20, and the primary and secondary devices 106, 108 used by the remote care providers 16 are also connected to the communications network 20. The communications network 20 can include any type of wired or wireless connections or any combinations thereof. Examples of wireless connections include broadband cellular network connections such as 4G or 5G.

A request from the caregiver 12 will go out to all remote care providers 16 who have chosen to receive notifications for the request type and who are part of the health care system of the clinical care environment 10. Advantageously, the consultations between the caregiver 12 and the remote care providers 16 are guided by the virtual care management application 110 to take the burden off the caregiver 12 to reach out to multiple care providers for a consultation. Instead, a request from the caregiver is sent to a plurality of remote care providers, and the remote care provider who accepts first gets connected to the caregiver who sent the request. This is achieved through combination of routing logic with a user activated interface. Advantageously, the virtual care management application 110 combines patient contextual data in a single application with communications and task management platforms.

Additionally, the virtual care management application 110 enables the remote care providers 16 to cover multiple facilities within the health care system. Also, the virtual care management application 110 enables the remote care providers 16 to select and change the type of notifications, request types, and facilities or units that they will receive notifications and virtual care requests on their devices from the virtual care management application 110.

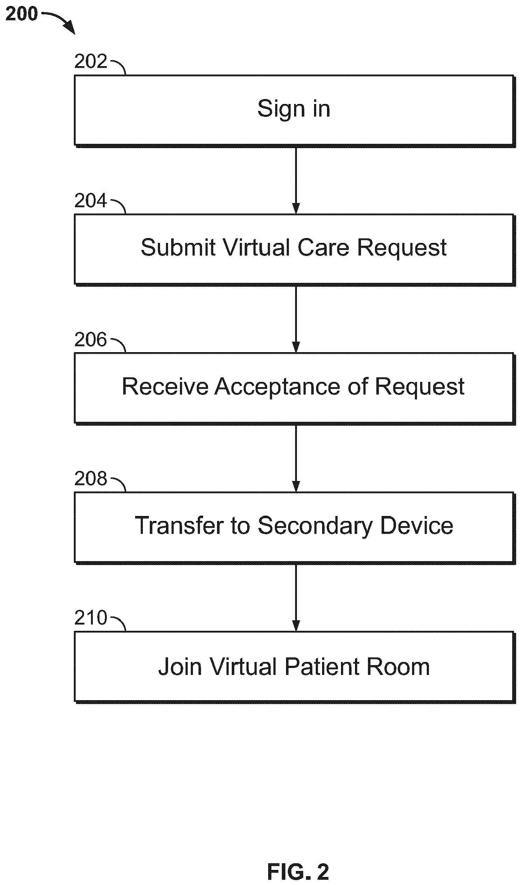

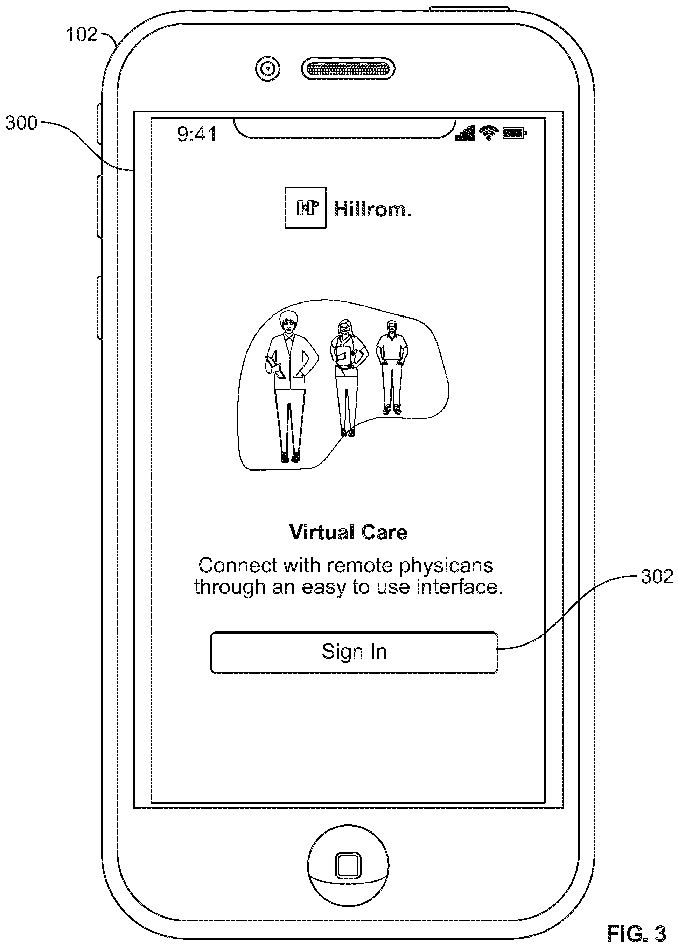

FIG. 2 illustrates an example of a method 200 of requesting a consultation with a remote care provider 16 using the virtual care management application 110 installed or otherwise accessible on the primary device 102 of the caregiver 12. The method 200 includes an operation 202 of signing into the virtual care management application 110, an operation 204 of submitting a virtual care request, an operation 206 of receiving an acceptance of the virtual care request from a remote care provider 16, an operation 208 of transferring the consultation to a secondary device, and an operation 210 of joining a virtual patient room. Each of the operations 202-210 will be described in more detail with respect to the various screens shown in FIGS. 3-26.

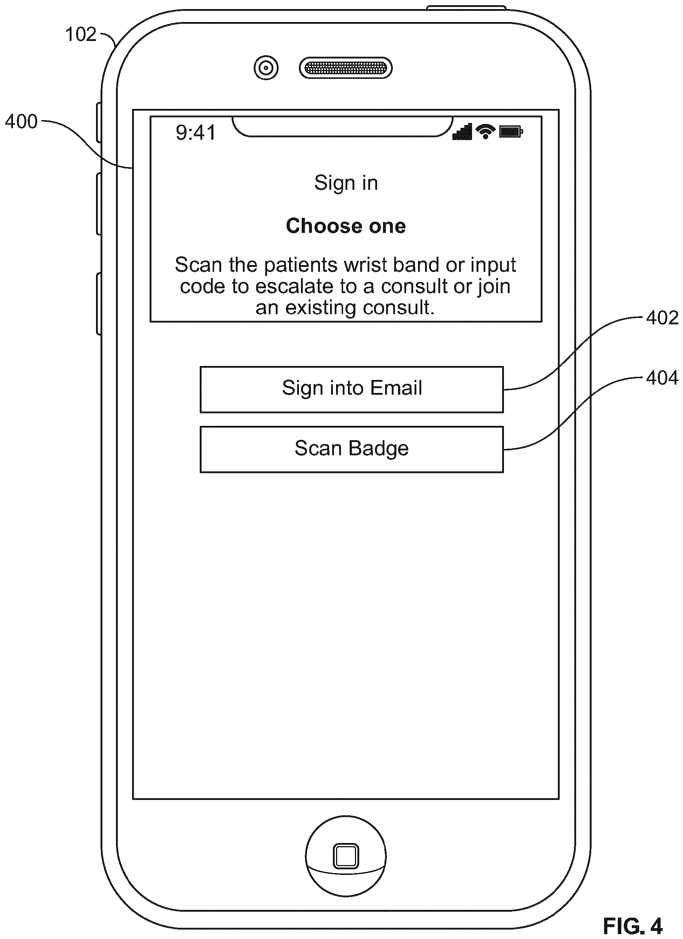

FIG. 3 illustrates an example of a sign in screen 300 generated on the primary device 102 of the caregiver 12 by the virtual care management application 110. The sign in screen 300 shown in FIG. 3 (as well as the sign in screens shown in FIGS. 4-6) can be used by the caregiver 12 to perform the operation 202 of signing into the virtual care management application 110, in accordance with the method 200 that is described above. The sign in screen 300 can be automatically displayed when the caregiver 12 opens the virtual care management application 110 on their primary device. The sign in screen 300 includes a sign in icon 302 that the caregiver 12 can select to sign into the virtual care management application 110.

FIG. 4 illustrates an example of a sign in screen 400 that is generated on the primary device 102 of the caregiver 12 by the virtual care management application 110 in response to the caregiver 12 selecting the sign in icon 302 of the sign in screen 300 shown in FIG. 3. The sign in screen 400 provides options for the caregiver 12 to sign in. For example, a first option 402 can be selected by the caregiver 12 to sign in using single sign-on (SSO) credentials, and a second option 404 can be selected by the caregiver 12 to sign in by scanning their badge.

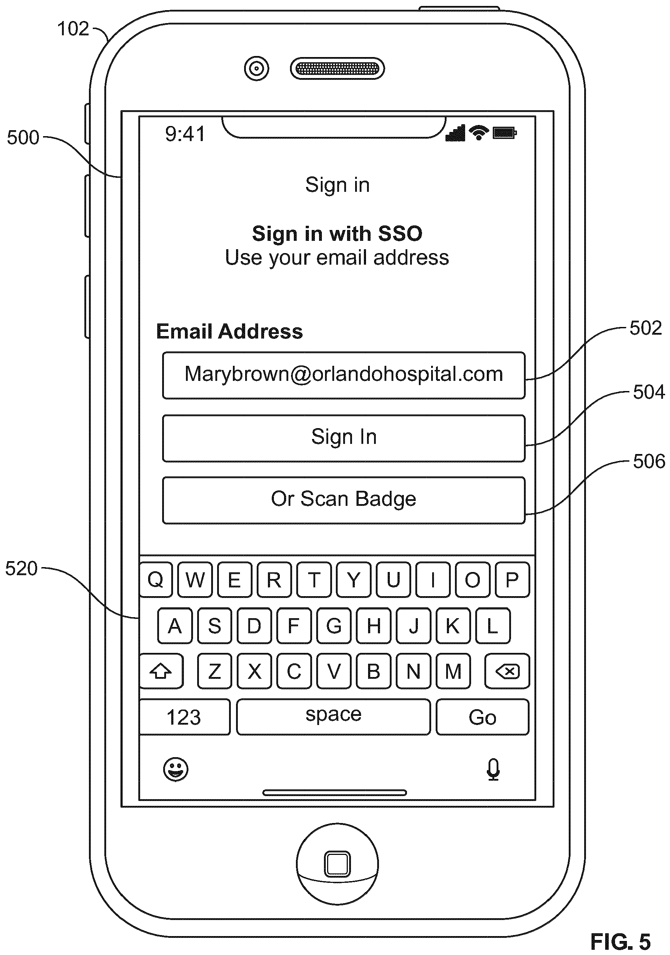

FIG. 5 illustrates an example of a sign in screen 500 generated on the primary device 102 of the caregiver 12 by the virtual care management application 110 in response to the caregiver 12 selecting the first option 402 of the sign in screen 400 shown in FIG. 4. In this example, the sign in screen 500 allows the caregiver 12 to sign into the virtual care management application 110 using single sign-on (SSO) such as by using a keyboard 520 to type into an email address field 502 an email address associated with related software applications accessible on the primary device 102 (or the secondary device 104). For example, the email address can be associated with a mobile caregiver application for managing messages between caregivers in a healthcare facility such as the one described in U.S. Provisional Patent Application No. 63/109,464, titled Managing Caregiver Messages, filed on November 4, 2020, and/or a family communications application for managing updates from the caregiver to one or more family members of the patient such as the one described in U.S. Provisional Patent Application No. 63/163,468, titled Caregiver and Patient Family Communications, filed on March 19, 2021, the entireties of which are hereby incorporated by reference.

Once an email address has been entered into the email address field 502, the caregiver 12 can select the sign in icon 504 to sign into the virtual care management application 110 using SSO. Alternatively, the caregiver 12 can select a scan badge icon 506 to sign into the virtual care management application 110 by scanning their badge, as will be described next.

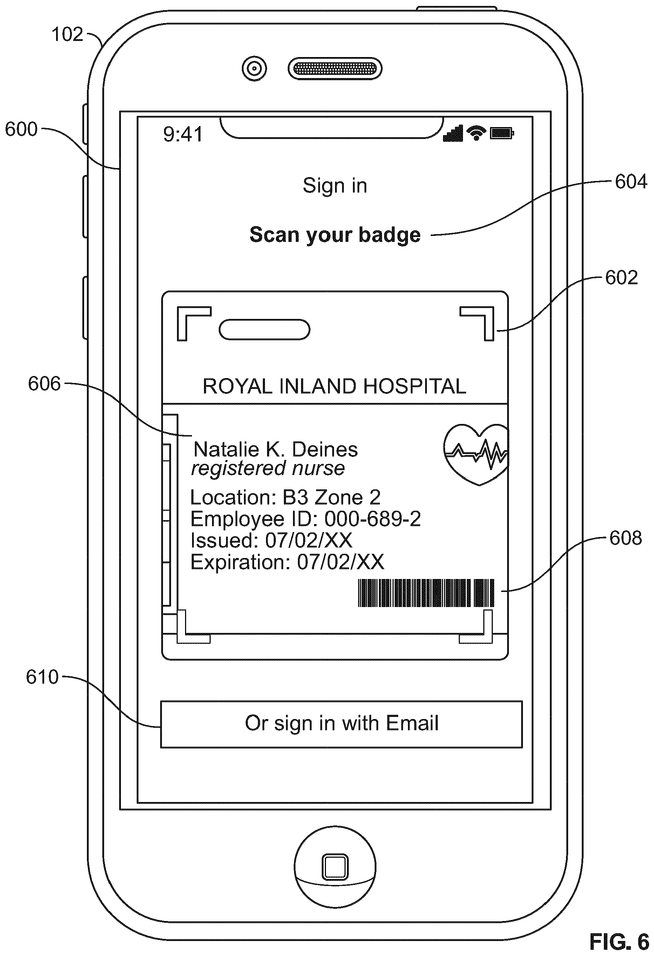

FIG. 6 illustrates another example of a sign in screen 600 that is generated on the primary device 102 of the caregiver 12 by the virtual care management application 110 in response to the caregiver 12 selecting the second option 404 of the sign in screen 400 of FIG. 4, or by selecting the scan badge icon 506 of the sign in screen 500 of FIG. 5. As shown in FIG. 6, the sign in screen 600 displays a frame 602 that surrounds an image feed from a camera of the primary device 102. The sign in screen 600 can include instructions 604 for the caregiver 12 to align the frame 602 with their badge 606 that includes machine-readable data 608 that identifies the caregiver 12. For example, the machine-readable data 608 can be a barcode, a QR code, or similar data representation that is recognizable by the camera of the primary device 102.

The virtual care management application 110 uses the camera of the primary device 102 (or the secondary device 104) to automatically detect and scan the machine-readable data 608 to sign in the caregiver 12 to the virtual care management application 110. In examples where the caregiver 12 is using the secondary device 104 as a workstation attached to a mobile cart, the caregiver 12 can use a handheld scanner to scan the machine-readable data 608 to sign in the caregiver 12 to the virtual care management application 110 on the workstation.

The sign in screen 600 can also have a sign in with email icon 610 that the caregiver 12 can select to sign into the virtual care management application 110 using single sign-on (SSO) credentials, such as described above. The virtual care management application 110 can connect back to a system of the clinical care environment 10 to validate that the caregiver 12 is authorized to access the virtual care management application 110. For example, the virtual care management application 110 can connect to an admission, discharge, and transfer (ADT) system or to a lightweight directory access protocol (LDAP) system to authenticate the credentials of the caregiver 12 for using the virtual care management application 110.

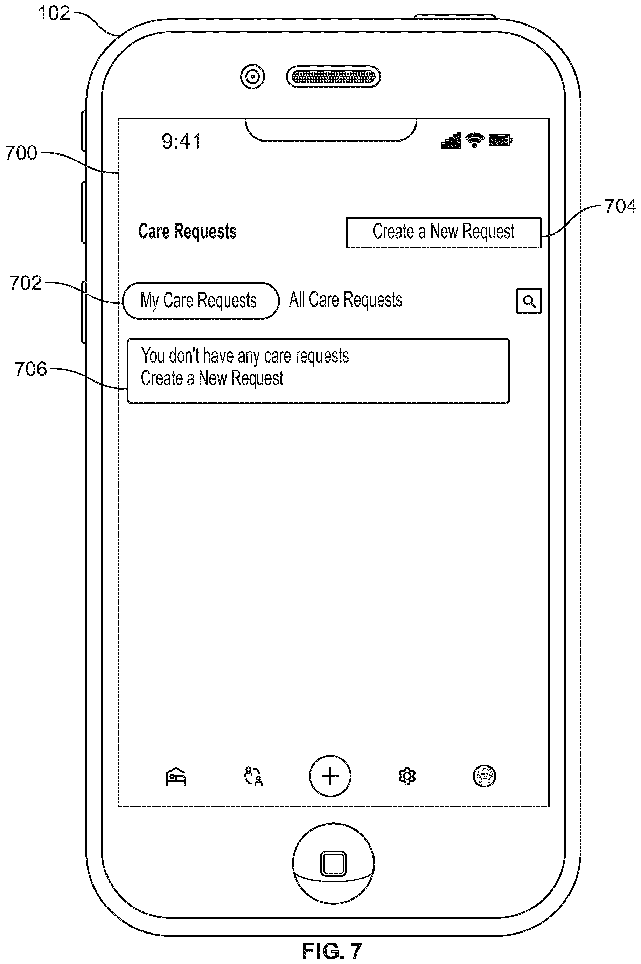

FIG. 7 illustrates an example of a care requests screen 700 that is generated on the primary device 102 upon the caregiver 12 successfully signing into the virtual care management application 110. The care requests screen 700 includes a my care requests tab 702 that can be selected by the caregiver 12 to display a list of pending care requests. In the example shown in FIG. 7, the care requests screen 700 includes a message 706 that the caregiver 12 does not have any care requests. The care requests screen 700 further includes a create new request icon 704 that the caregiver 12 can select to create a new care request. This feature will be described next.

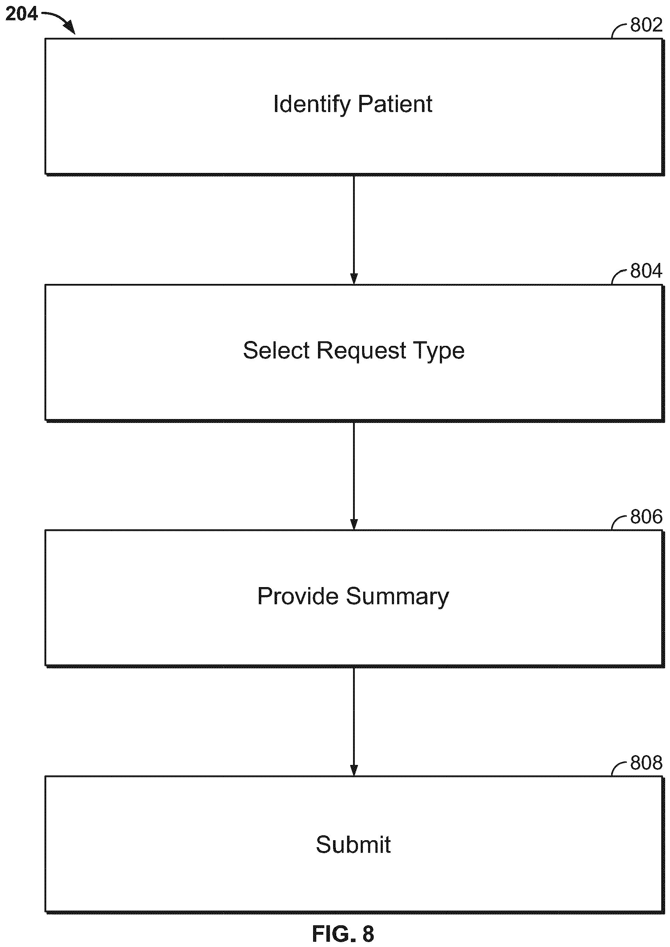

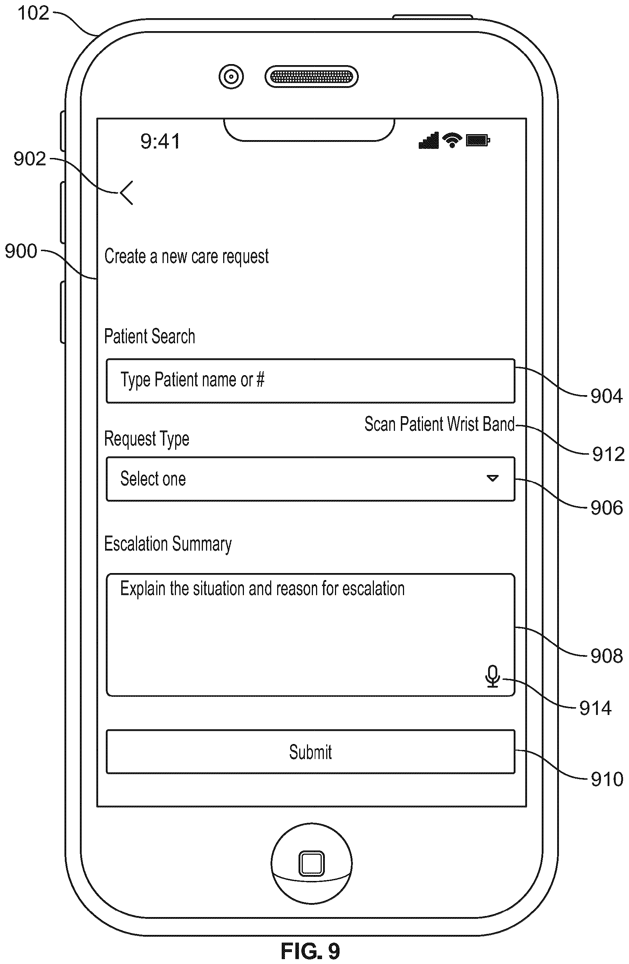

FIG. 8 illustrates in more detail the operation 204 of submitting a virtual care request that is part of the method 200 of FIG. 2. As shown in FIG. 8, submitting a virtual care request includes a step 802 of identifying a patient, a step 804 of selecting a request type, a step 806 of providing a summary to explain the request, and a step 808 of submitting the virtual care request. Each of these steps will now be described in more detail with reference to FIGS. 9-15.

FIG. 9 illustrates an example of a create new care request screen 900 that is generated on the primary device 102 of the caregiver 12 by the virtual care management application 110 in response to the caregiver 12 selecting the create new request icon 704 of the care requests screen 700 of FIG. 7. The create new care request screen 900 has a return icon 902 that the caregiver 12 can select to return back to the care requests screen 700 of FIG. 7. The create new care request screen 900 further includes an identify patient section 904, an identify request type section 906, a summary input section 908, and a submit icon 910. A link 912 is provided under the identify patient section 904 that can be selected to identify the patient 14 by scanning a patient label.

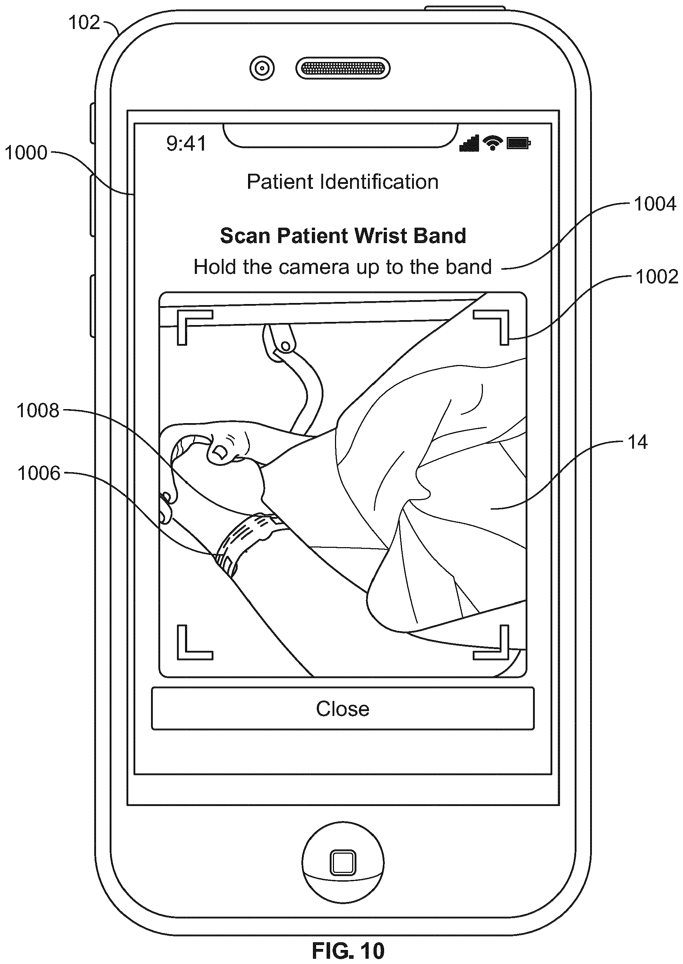

FIG. 10 illustrates an example of a patient identification screen 1000 that is generated on the primary device 102 of the caregiver 12 by the virtual care management application 110 in response to the caregiver 12 selecting the link 912 from the create new care request screen 900 of FIG. 9. The patient identification screen 1000 can be used by the caregiver 12 to perform the step 802 of identifying the patient shown in FIG. 8.

The patient identification screen 1000 displays a frame 1002 that surrounds an image feed from the camera of the primary device 102, and can include instructions 1004 for the caregiver 12 to align the frame 1002 with a label 1006 that includes machine-readable data 1008 that identifies the patient 14. For example, the machine-readable data 1008 can be a barcode, a QR code, or similar data representation recognizable by the camera of the primary device 102. In some examples, the label 1006 is on a bracelet worn by the patient 14.

The virtual care management application 110 uses the camera of the primary device 102 to automatically detect and scan the machine-readable data 1008 to identify the patient 14. In alternative examples, such as when the caregiver 12 is using the secondary device 104, the caregiver 12 can use a camera of the secondary device 104 (e.g., when the secondary device 104 is a tablet computer) or can use a handheld scanner (e.g., when the secondary device 104 is a workstation attached to a mobile cart) to scan the machine-readable data 1008 to identify the patient 14. This allows for quick identification of the patient 14 and also reduces errors. This scan will also trigger a validation and retrieval where patient demographic data will be returned to ensure the right patient has been identified. This may include the patient's date of birth, gender, name, medical record number (MRN), and the like.

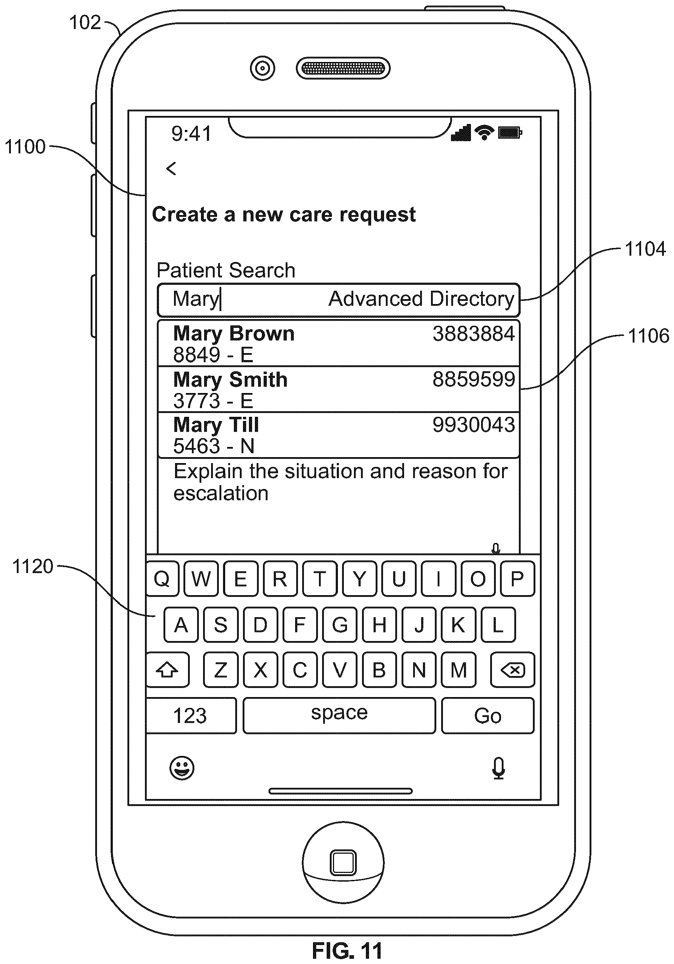

FIG. 11 illustrates an example of a create new care request screen 1100 that can also be used by the caregiver 12 to perform the step 802 of identifying the patient. Instead of scanning a label worn by the patient 14, the create new care request screen 1100 allows the caregiver 12 to begin typing into an identify patient section 1104 the patient's name or medical record number, and the virtual care management application 110 can populate a list of patients 1106 from which the caregiver 12 can select to identify the patient 14. For example, the caregiver 12 can type "Mary" using a keyboard 1120, and in response, the virtual care management application 110 generates a list of patients 1106 (e.g., "Mary Brown", "Mary Smith", "Mary Till", etc.) from which the caregiver 12 can select to enter the patient 14 into the identify patient section 1104.

Step 802 of identifying the patient can further include additional security measures to ensure that the patient 14, the caregiver 12, and patient data (e.g., vital signs, lab results, and other information acquired from an electronic medical record (EMR) of the patient 14 or from one or more monitoring devices connected to the patient 14), are correctly combined or otherwise aligned for creating a virtual care request. In some examples, data from a real-time locating system (RTLS) is used to verify that the correct patient, the correct caregiver, and the correct patient data are combined or otherwise aligned for creating a virtual care request.

As an illustrative example, RTLS data can be used to confirm the identity of the patient 14 identified in step 802 by identifying a tag worn by the patient 14, or a tag attached to a device associated with the patient 14 such as a hospital bed, vital signs spot monitor, smart TV, or tablet computer (e.g., an experience pod from Hillrom^{®}). When a tag worn by the patient 14, or a tag attached to a device associated with the patient 14 matches the identity of the patient 14 acquired from step 802, the identity of the patient 14 is confirmed. Otherwise, when a tag worn by the patient 14, or a tag attached to a device associated with the patient 14 does not match the identity of the patient 14 acquired from step 802, the method 200 terminates.

In this example, the RTLS data provides an automated multifactor process for confirming that the patient 14 has been correctly identified in step 802, and can reduce errors that result from creating a virtual care request for the wrong patient. This can help ensure compliance with the Health Insurance Portability and Accountability Act of 1996 (HIPAA), which is a federal law that requires the creation of national standards to protect sensitive patient health information from being disclosed without the patient's consent or knowledge.

As another illustrative example, the RTLS data can also be used to confirm the identity of the caregiver 12 who creates the virtual care request for the patient 14. For example, the RTLS data can allow the caregiver 12 to have a single sign into the virtual care management application 110 installed or otherwise accessible on the primary device 102 of the caregiver 12. Once the caregiver 12 is identified from using the RTLS data, the caregiver 12 can automatically be identified as the requestor or creator of the virtual care request.

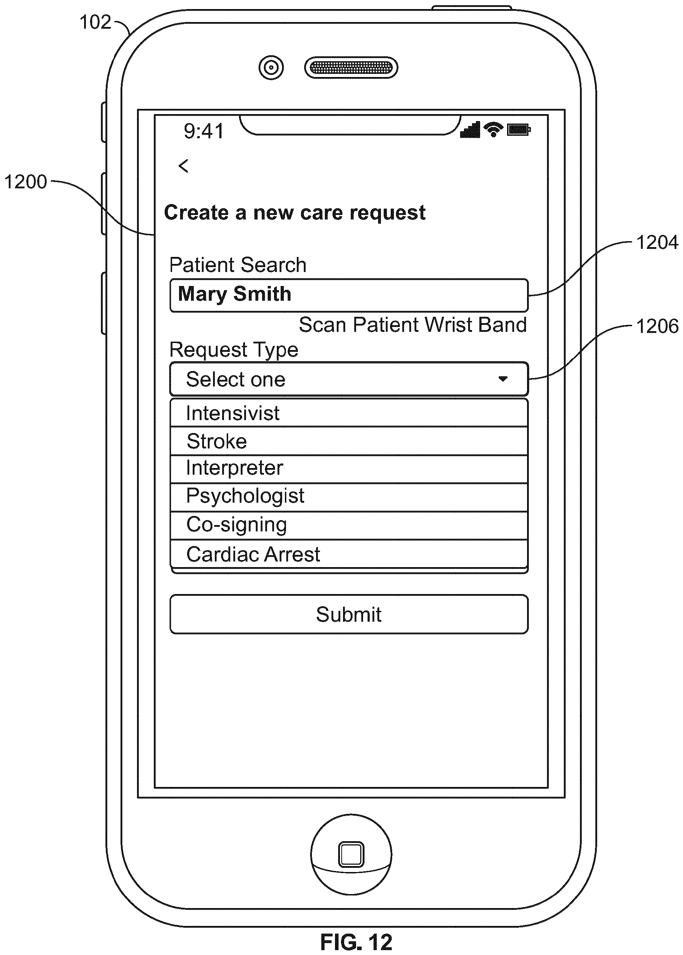

FIG. 12 illustrates an example of a create new care request screen 1200 that is generated on the primary device 102 of the caregiver 12 by the virtual care management application 110 after a patient is entered in an identify patient section 1204. The create new care request screen 1200 includes an identify request type section 1206 that can be used by the caregiver 12 to perform the step 804 of selecting a request type. The request type may be configured by specialty or by condition (e.g., TeleStroke). In this example, the identify request type section 1206 is a drop down menu that provides a plurality of request types for the caregiver 12 to select. In this example, the request types correspond to a remote care provider, and the caregiver 12 can select from the drop down menu in the identify request type section 1206 an intensivist, a neurologist, a cardiologist, a psychologist, an interpreter, and the like.

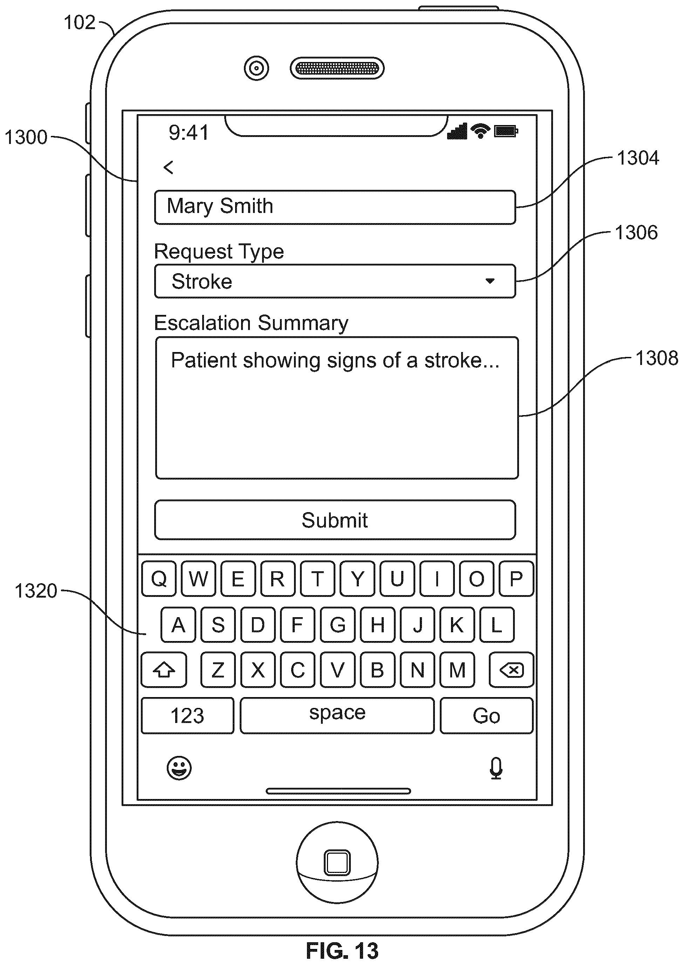

FIG. 13 illustrates an example of a create new care request screen 1300 that is generated on the primary device 102 of the caregiver 12 by the virtual care management application 110. The create new care request screen 1300 includes an identify patient section 1304 that includes a patient identified from step 802 of FIG. 8, an identify request type section 1306 that includes a stroke specialist selected in step 804 of FIG. 8, and a summary input section 1308 into which the caregiver 12 can enter a summary of the virtual care request in accordance with the step 806 of FIG. 8. In the example shown in FIG. 13, the caregiver 12 can use a keyboard 1320 to type the summary of the virtual care request into the summary input section 1308. Alternatively, the summary input section 1308 can include a microphone icon (such as the microphone icon 914 shown in FIG. 9), that the caregiver 12 can select for voice dictation to enter the summary of the virtual care request into the summary input section 1308.

In some examples, an urgency level (e.g., "High", "Moderate", or "Low") can be tethered to the request type as configured by the administrator. In other examples, the caregiver 12 can add an urgency level to the virtual care request. In some further examples, the caregiver 12 can include risk scores in the virtual care request to help focus on the reason for escalation. The risk scores can be based on the patient's demographics and diagnoses, and can be used to quantify the likelihood that the patient will experience a deterioration in condition.

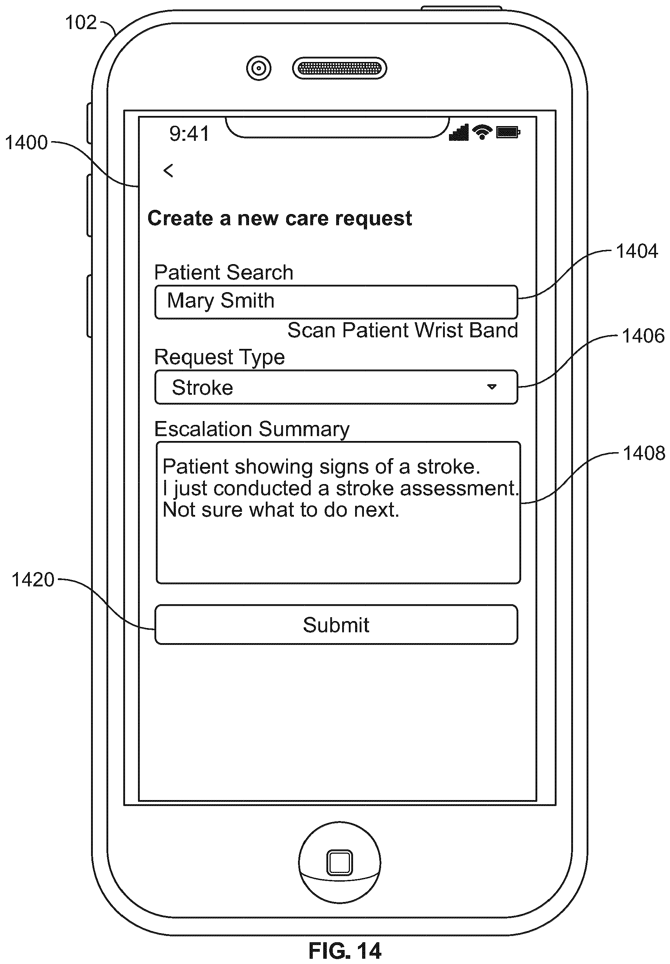

FIG. 14 illustrates an example of a create new care request screen 1400 generated on the primary device 102 of the caregiver 12 by the virtual care management application 110. The create new care request screen 1400 has an identify patient section 1404 that includes a patient name identified from step 802 of FIG. 8, an identify request type section 1406 that includes a stroke specialist selected in step 804 of FIG. 8, a summary input section 1408 that includes a summary entered in accordance with step 806 of FIG. 8, and a submit icon 1410 that can be selected by the caregiver 12 to perform the step 808 of submitting the virtual care request.

Once the virtual care request is submitted, it is routed through the communications network 20 to one or more remote care providers 16. The virtual care management application 110 utilizes intelligent routing to send the virtual care request to a remote care provider 16 who is logged in to the virtual care management application 110 within their health care system and who has selected to receive notifications for the request type of the virtual care request.

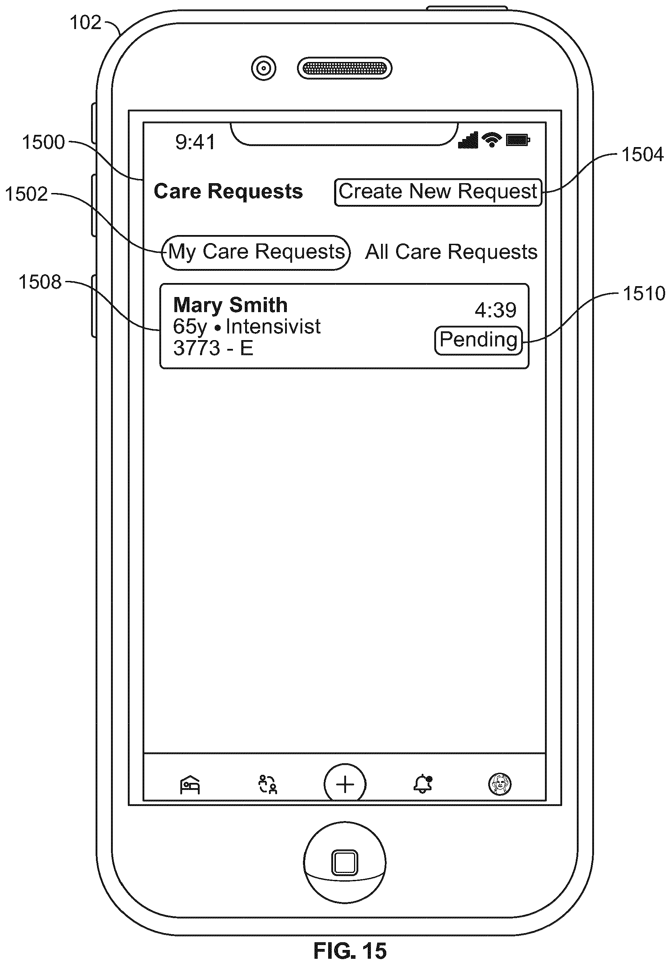

FIG. 15 illustrates an example of a care requests screen 1500 that is generated on the primary device 102 of the caregiver 12 by the virtual care management application 110 after the caregiver 12 selects the submit icon 1410 from the create new care request screen 1400 of FIG. 14. As shown in FIG. 15, a my care requests tab 1502 can include a care request 1508 submitted in accordance with steps 802-808 shown in FIG. 8, and in accordance with the screens shown in FIG. 9-14. The care requests screen 1500 also has a create new request icon 1504 that the caregiver 12 can select to create another new care request.

The care request 1508 can include information that identifies the patient such as the patient's name (e.g., "Mary Smith"), the patient's age (e.g., "65y"), the patient's medical record number (e.g., "3773 - E"), and the like. The care request 1508 can also include the type of request (e.g., "Intensivist"), and the time the care request was sent (e.g., "4:39"). Also, the caregiver 12 can select the care request 1508 to expand it and view additional details.

In some examples, the care request 1508 can include a timer that is configurable based on the request type. For example, when the request type is for a stroke, the care request 1508 can display a visual indicator such as a countdown clock that sets a maximum limit for how long the caregiver 12 can wait before taking clinical action to minimize patient deterioration.

The care request 1508 further includes a status box 1510 that displays a status of the care request 1508. In FIG. 15, the status box 1510 displays that the care request 1508 is "Pending". In some examples, the status box 1510 is color coded to further indicate the status of the care request 1508. For example, the status box 1510 can be colored red to indicate a pending care request, and can transition to being colored green when the care request is accepted.

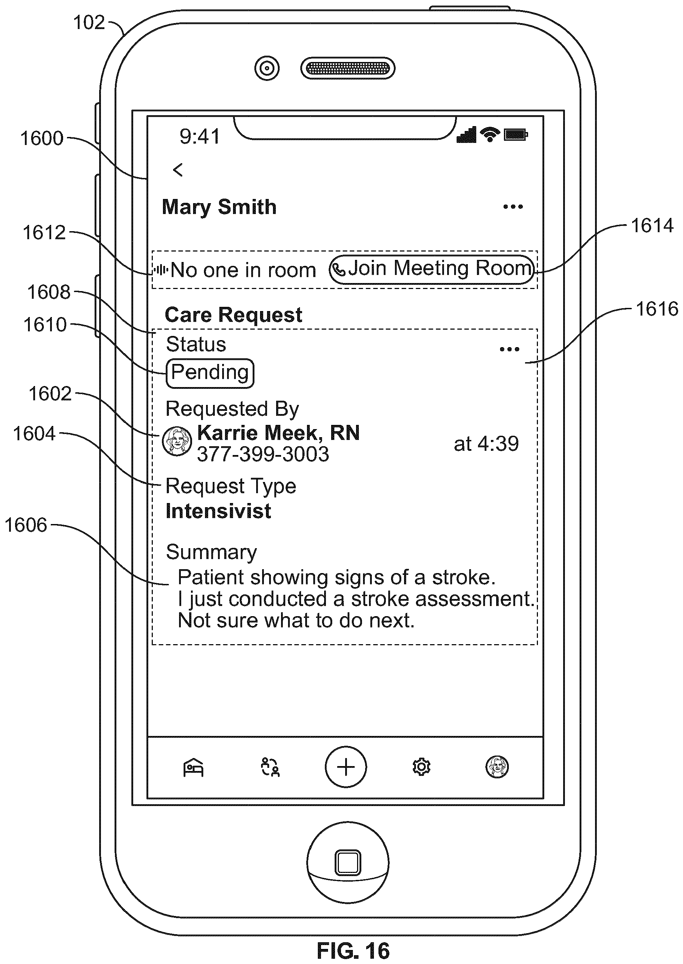

FIG. 16 illustrates an example of a care request screen 1600 generated on the primary device 102 of the caregiver 12 by the virtual care management application 110 in response to the caregiver 12 selecting the care request 1508 from FIG. 15. The care request screen 1600 can display additional details of a care request 1608 such as information 1602 that identifies the caregiver 12 or the person who submitted the care request (e.g., the person's name, position, and telephone number), the type of request 1604 (e.g., Intensivist"), and a summary 1606 of the care request 1608, as well as a status box 1610 for displaying a status of the care request 1608.

The care request screen 1600 includes a meeting room module 1612 that indicates whether a remote care provider has joined a virtual patient room (i.e., in response to accepting the care request 1608), and a join meeting room icon 1614 that the caregiver 12 can select to enter the virtual patient room for a consultation with the remote care provider. In the example of FIG. 16, the status box 1610 indicates that the care request 1608 is pending, and the meeting room module 1612 indicates that no one has joined the virtual patient room. The caregiver 12 can select an edit icon 1616 to edit or cancel the care request 1608.

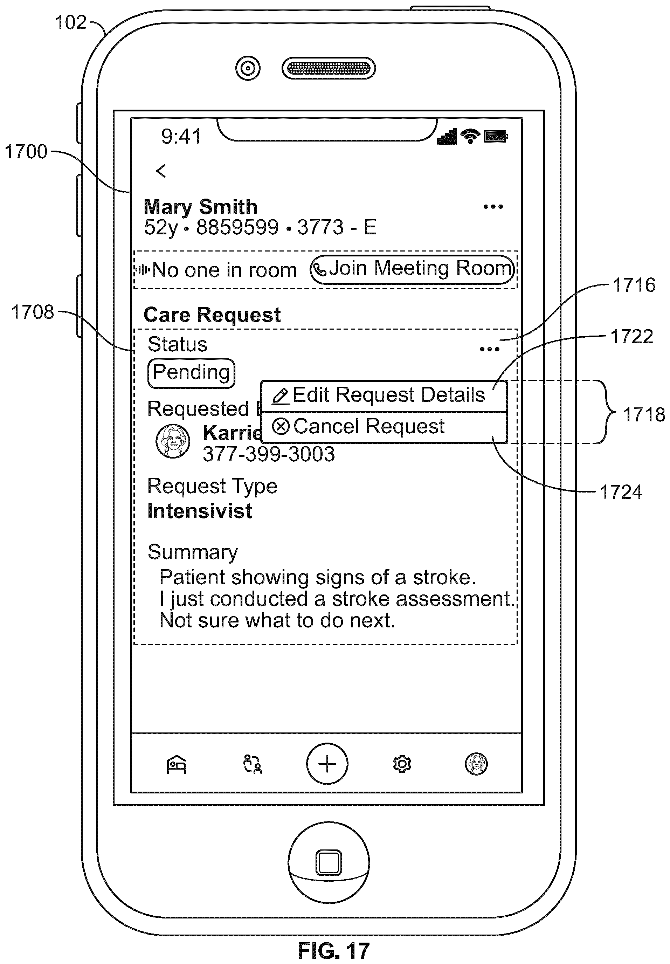

FIG. 17 illustrates an example of a care request screen 1700 that includes an edit tab 1718 generated in response to the caregiver 12 selecting an edit icon 1716. The edit tab 1718 includes an edit option 1722 that the caregiver 12 can select to edit a care request 1708. For example, the caregiver 12 can edit the care request 1708 to provide updated information in the summary, or to make other changes to the care request 1708. Also, the edit tab 1718 includes a cancel option 1724 that the caregiver 12 can select to cancel the care request 1708.

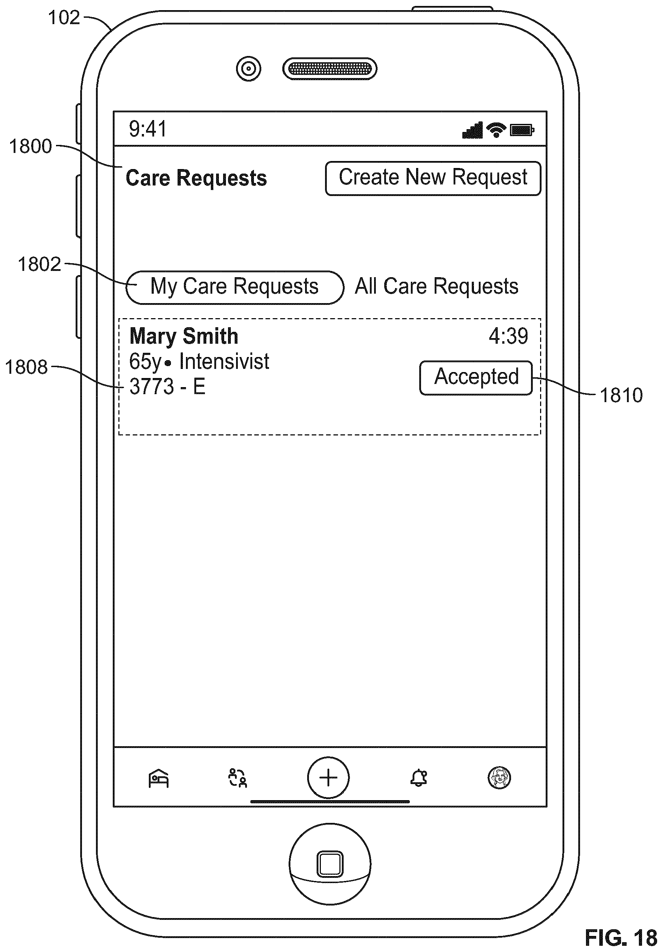

FIG. 18 illustrates an example of a care requests screen 1800 that is generated on the primary device 102 of the caregiver 12 by the virtual care management application 110. In this example, a my care requests tab 1802 includes a care request 1808 that was submitted in accordance with steps 802-808 shown in FIG. 8, and in accordance with the screens shown in FIG. 9-14. In this example, the care request 1808 has been accepted. For example, a status box 1810 displays that the care request 1808 is "Accepted". In some examples, the status box 1810 is colored green to indicate that the care request 1808 has been accepted.

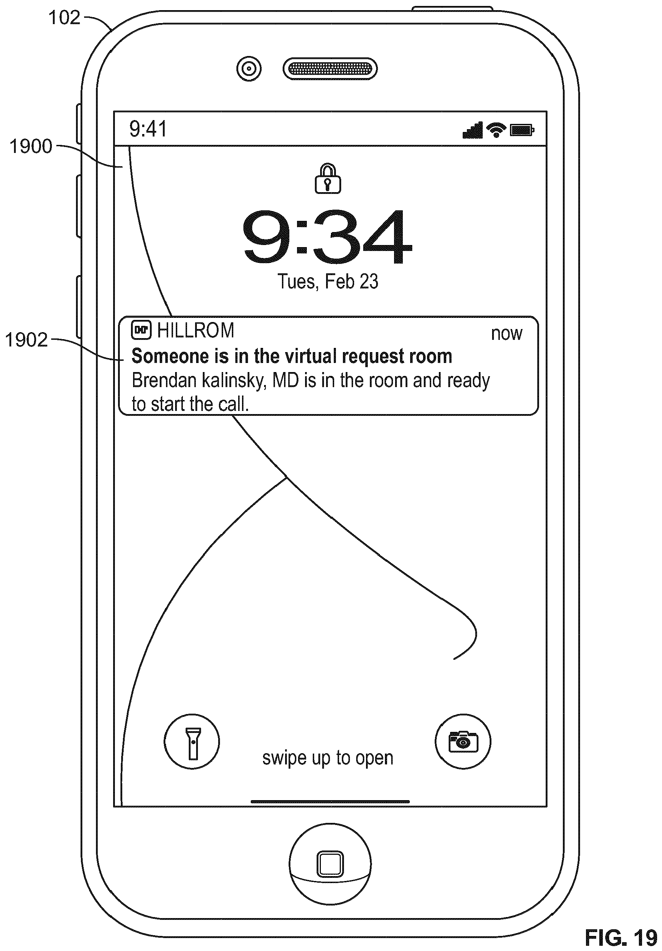

FIG. 19 illustrates an example of a notification 1902 that is generated on the primary device 102 of the caregiver 12 by the virtual care management application 110. As shown in FIG. 19, the notification 1902 is displayed on a lock screen 1900 of the primary device 102. The notification 1902 includes a message regarding the status of a submitted care request such as whether the care request was accepted or whether a remote care provider has joined a virtual patient room in response to the care request. Upon receiving the notification 1902 on the primary device 102, the caregiver 12 can open the virtual care management application 110 such as to join the virtual patient room with the remote care provider.

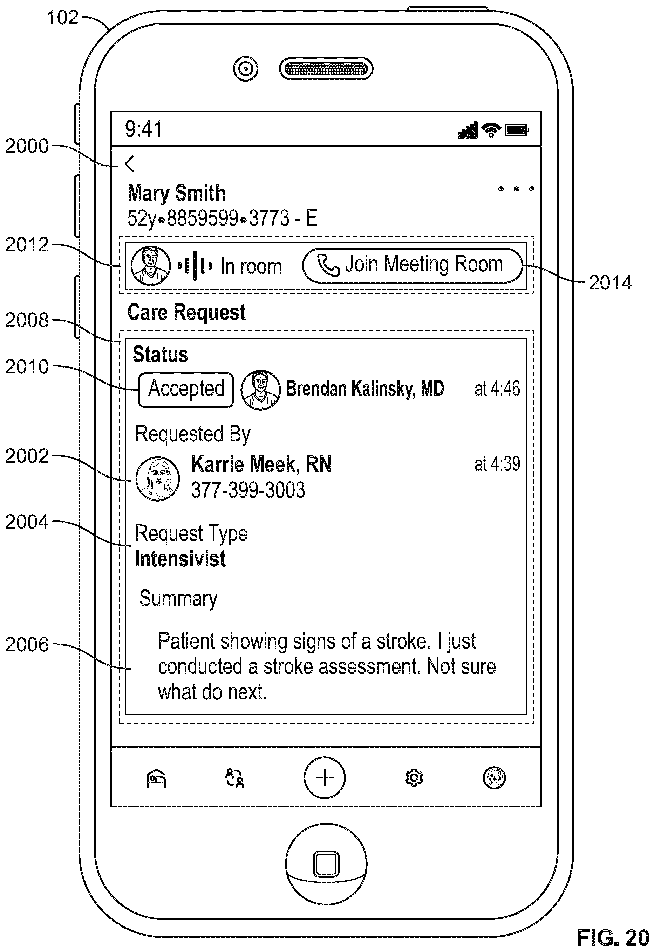

FIG. 20 illustrates an example of a care request screen 2000 generated on the primary device 102 of the caregiver 12 by the virtual care management application 110. In this example, the care request screen 2000 displays a care request 2008, and resembles the care request screen 1600 of FIG. 16. For example, the care request 2008 includes information 2002 that identifies the caregiver 12 or the person who submitted the care request (e.g., the person's name, position, and telephone number), the type of request 2004 (e.g., "Intensivist"), a summary 2006 of the care request 2008, and a status box 2010 for displaying a status of the care request 2008.

In the illustrative example shown in FIG. 20, the status box 2010 indicates that the care request 2008 has been "Accepted" by a remote care provider. As described above, the status box 2010 can be colored green to indicate that the care request 2008 has been accepted. In some examples, the name of the remote care provider who accepted the care request 2008 can also be displayed next to the status box 2010. In some further examples, the time that the care request 2008 was accepted by the remote care provider can also be displayed.

The care request screen 2000 further includes a meeting room module 2012 that indicates that the remote care provider is in the virtual patient room, and that includes a join meeting room icon 2014. The caregiver 12 can select the join meeting room icon 2014 to enter the virtual patient room for a consultation with the remote care provider. By selecting the join meeting room icon 2014, the caregiver 12 performs operation 210 of the method 200 of FIG. 2.

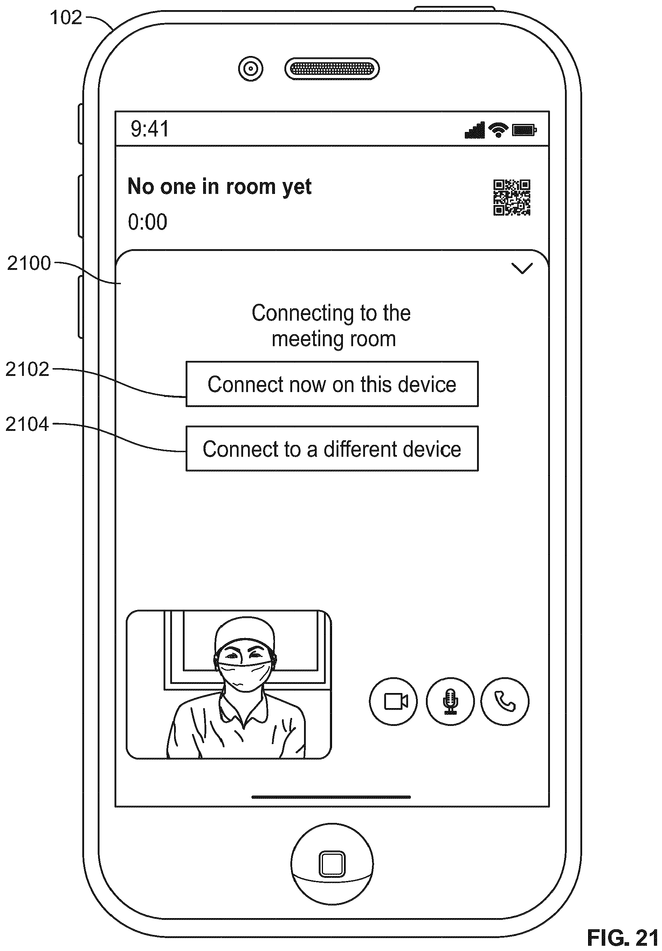

FIG. 21 illustrates an example of a connection screen 2100 generated on the primary device 102 of the caregiver 12 by the virtual care management application 110 in response to the caregiver 12 selecting the join meeting room icon 1614 from FIG. 16. The connection screen 2100 prompts the caregiver 12 to select a first connection option 2102 that allows the caregiver 12 to join the virtual patient room using the primary device 102, or a second connection option 2104 that allows the caregiver 12 to join the virtual patient room using the secondary device 104 (see FIG. 1). The first and second connection options will now be described in more detail.

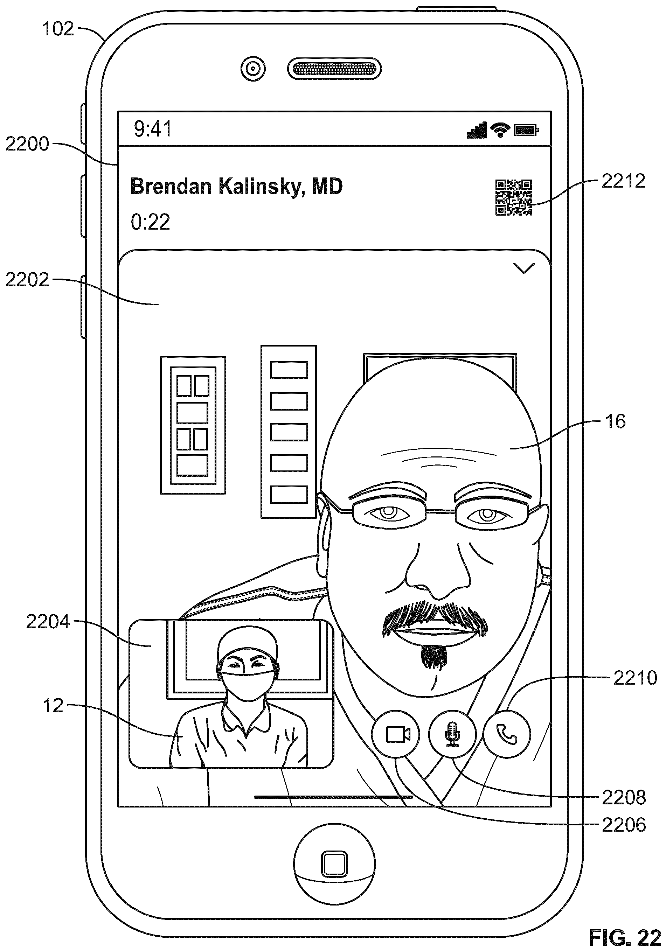

FIG. 22 illustrates an example of a meeting room screen 2200 that is generated on the primary device 102 of the caregiver 12 by the virtual care management application 110 in response to the caregiver 12 selecting the first connection option 2102 from the connection screen 2100 of FIG. 21. The meeting room screen 2200 can display the name of the remote care provider 16 and can also display a duration of the meeting. The meeting room screen 2200 further includes a window 2202 that displays a live video feed of the remote care provider 16 who accepted the care request. The caregiver 12 can communicate with the remote care provider 16 using the audio system of the primary device 102 (i.e., speakers and microphone), while viewing the remote care provider 16 in the window 2202 such that the meeting room screen 2200 provides at least a one-way video conference with the remote care provider 16.

The meeting room screen 2200 can further include a window 2204 that displays a live video feed of the caregiver 12 acquired from the camera of the primary device 102. In such examples, the meeting room screen 2200 can provide a two-way video conference between the caregiver 12 and remote care provider 16. The meeting room screen 2200 can include a video camera icon 2206 that the caregiver can select to turn on and off the camera of the primary device 102, and thereby allow or block the live video feed of the caregiver 12. The meeting room screen 2200 can also include a microphone icon 2208 that the caregiver 12 can select to turn off and on the microphone of the primary device 102, and thereby mute and unmute the caregiver 12. The meeting room screen 2200 can also include a hang up icon 2210 that the caregiver 12 can select to terminate the video conference with the remote care provider 16.

In some examples, the meeting room screen 2200 can further include a link 2212 that the caregiver 12 can select during the consultation with the remote care provider 16 to transfer the consultation to another device such as the secondary device 104 of the caregiver 12.

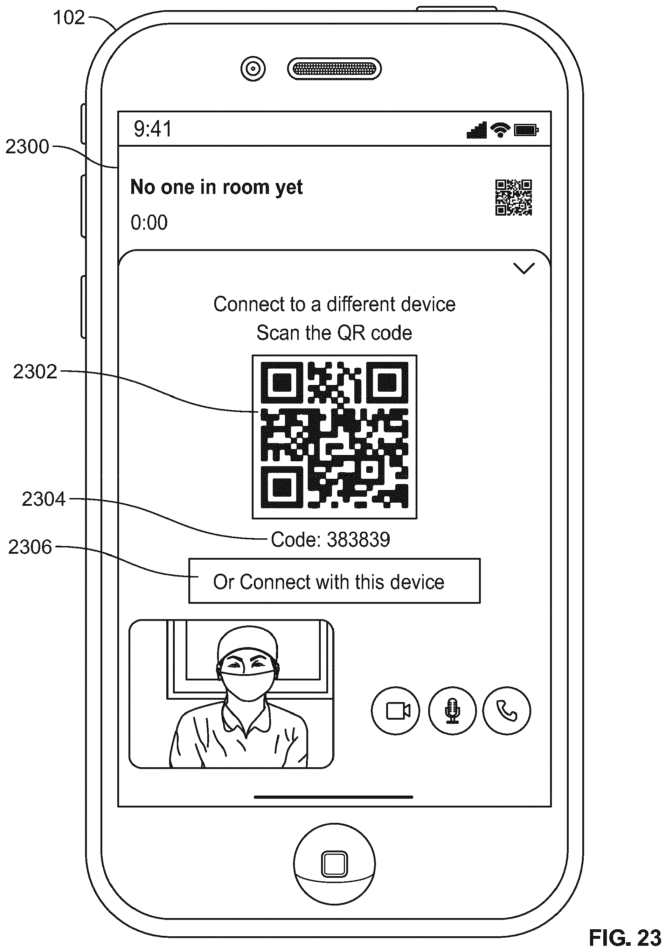

FIG. 23 illustrates an example of a connection transfer screen 2300 that is generated on the primary device 102 of the caregiver 12 by the virtual care management application 110 in response to the caregiver 12 selecting the second connection option 2104 from the connection screen 2100 of FIG. 21. The connection transfer screen 2300 displays machine-readable data 2302 that identifies the virtual patient room that the remote care provider has joined. The machine-readable data 2302 can be used to transfer the virtual patient room with the remote care provider to another device such as the secondary device 104. For example, the machine-readable data 2302 can be a QR code that is recognizable by a camera of the secondary device 104, and that can be used to transfer the virtual patient room to the secondary device 104.

Alternatively, or in addition to displaying the machine-readable data 2302, the connection transfer screen 2300 can also display a code 2304 that can be manually entered by caregiver 12 to transfer the virtual patient room with the remote care provider 16 to the secondary device 104. In further examples, the connection transfer screen 2300 can display an icon or link that the caregiver 12 can select to send an SMS message or email to the secondary device 104 with an invitation to join the virtual patient room on the secondary device 104. The connection transfer screen 2300 can also include an icon 2306 that the caregiver 12 can select to join the virtual patient room on the primary device 102, and to display a meeting room screen with the remote care provider such as the one described above with reference to FIG. 22.

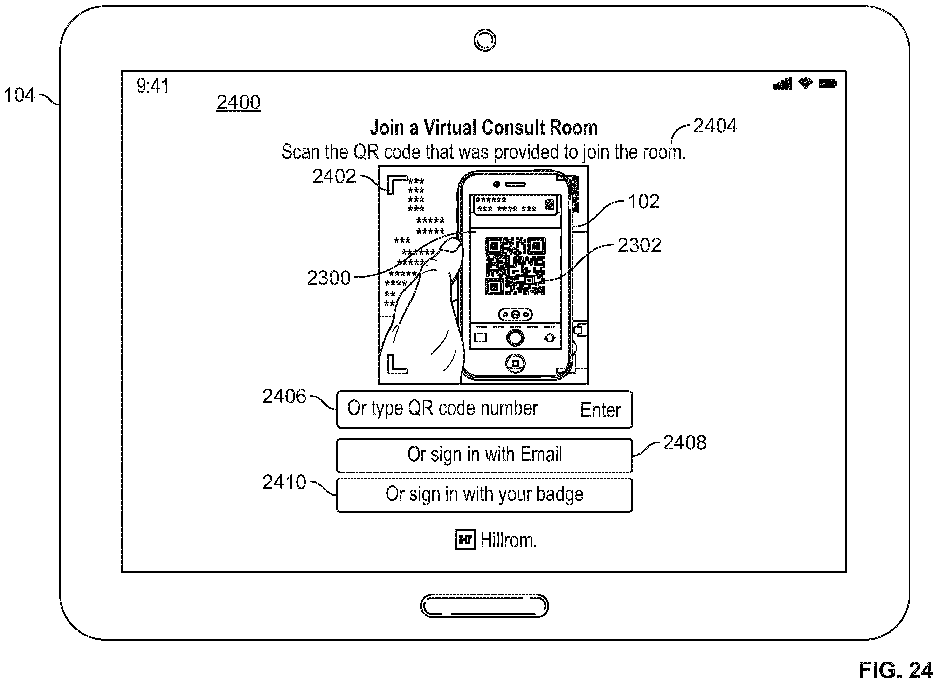

FIG. 24 illustrates an example of a connection transfer screen 2400 that is generated on the secondary device 104 of the caregiver 12 by the virtual care management application 110 when installed on the secondary device 104. As shown in FIG. 24, the connection transfer screen 2400 displays a frame 2402 that surrounds an image feed from a camera of the secondary device 104. As described above, in some examples the secondary device 104 can be a tablet computer. The connection transfer screen 2400 can include instructions 2404 for the caregiver 12 to align the frame 2402 with the connection transfer screen 2300 displayed on the primary device 102 that includes the machine-readable data 2302. The virtual care management application 110 uses the camera of the secondary device 104 to automatically detect and scan the machine-readable data 2302 to allow the caregiver 12 to join the virtual patient room on the secondary device 104.

Also, the connection transfer screen 2400 can include a code field 2406 that the caregiver 12 can use to manually enter the code 2304 from the connection transfer screen 2300 displayed on the primary device 102 for the caregiver 12 to join the virtual patient room on the secondary device 104. The connection transfer screen 2400 can also provide a sign in with email icon 2408 that the caregiver 12 can select to sign into the virtual care management application 110 on the secondary device 104 using single sign-on (SSO) credentials, such as in accordance with the example described above with respect to the primary device 102. Additionally, the connection transfer screen 2400 can also provide a sign in with your badge icon 2410 that the caregiver 12 can select to sign into the virtual care management application 110 on the secondary device 104 by scanning their badge with a camera of the secondary device 104, such as in accordance with the example described above with respect to the primary device 102.

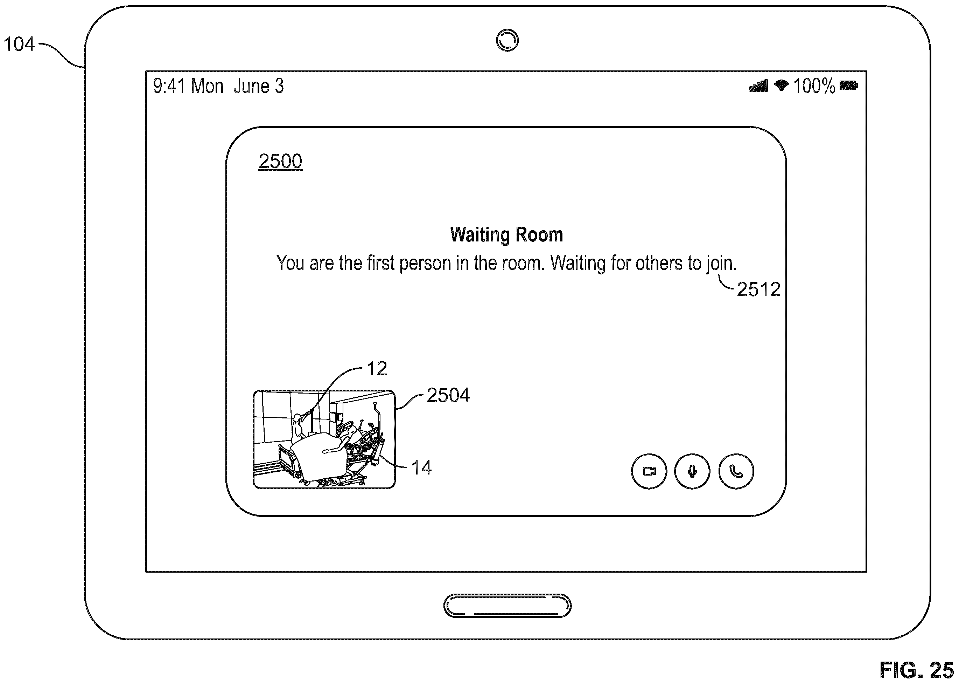

FIG. 25 illustrates an example of a meeting room screen 2500 that is generated on the secondary device 104 of the caregiver 12 by the virtual care management application 110 after the virtual patient room has been successfully transferred from the primary device 102 to the secondary device 104. In this example, the remote care provider has not yet joined the virtual patient room. The meeting room screen 2500 can include a message 2512 such as "You are the first person in the room. Waiting for others to join." In this example, the meeting room screen 2500 includes a window 2504 showing a live video feed of the caregiver 12 and patient 14.

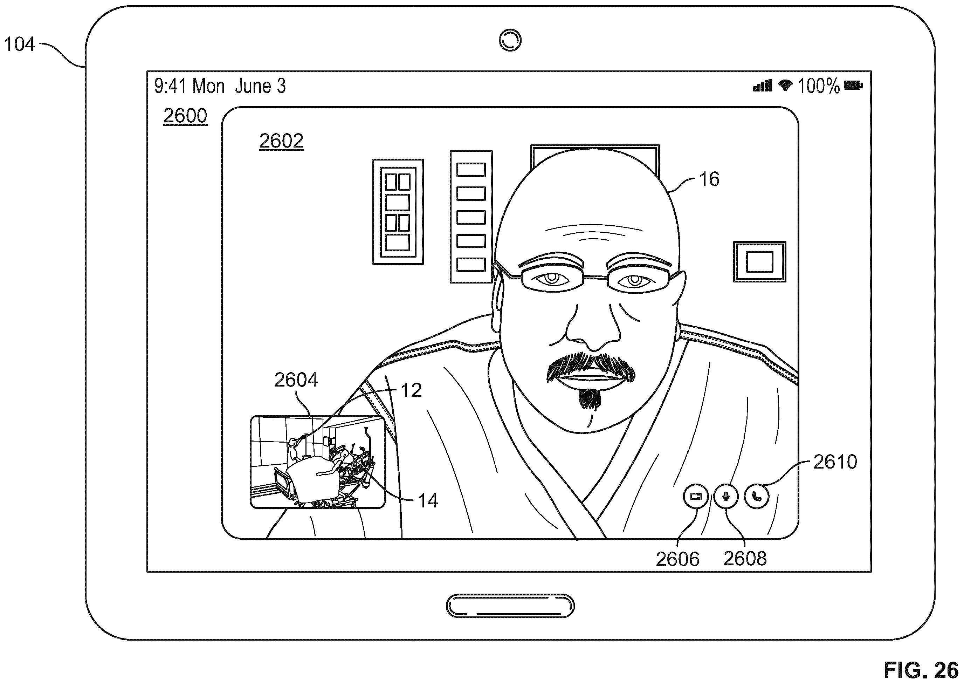

FIG. 26 illustrates another example of a meeting room screen 2600 that is generated on the secondary device 104 of the caregiver 12 by the virtual care management application 110 after the remote care provider 16 has joined the virtual patient room. The meeting room screen 2600 is similar to the one described on the primary device 102 in FIG. 22. For example, the meeting room screen 2600 includes a window 2602 that displays a live video feed of the remote care provider 16 who accepted the care request. The caregiver 12 can communicate with the remote care provider 16 using the audio system of the secondary device 104 (i.e., speakers and microphone), while viewing the remote care provider 16 in the window 2602.

The meeting room screen 2600 can further include a window 2604 that displays a live video feed of the caregiver 12 and patient 14 acquired from the camera of the secondary device 104. Thus, in some examples, the meeting room screen 2600 can provide a two-way video conference between the caregiver 12 and remote care provider 16.

As shown in FIG. 26, the meeting room screen 2600 has a video camera icon 2606 that the caregiver can select to turn on and off the camera of the secondary device 104 to allow or block the live video feed of the caregiver 12 inside the window 2604. The meeting room screen 2600 can also include a microphone icon 2608 that the caregiver 12 can select to turn off and on the microphone of the secondary device 104, and thereby mute and unmute the caregiver 12. The meeting room screen 2600 also includes a hang up icon 2610 that the caregiver 12 can select to terminate the video conference with the remote care provider 16.

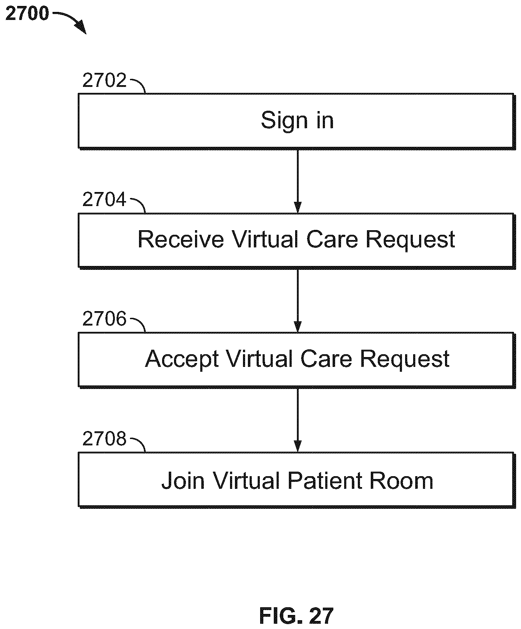

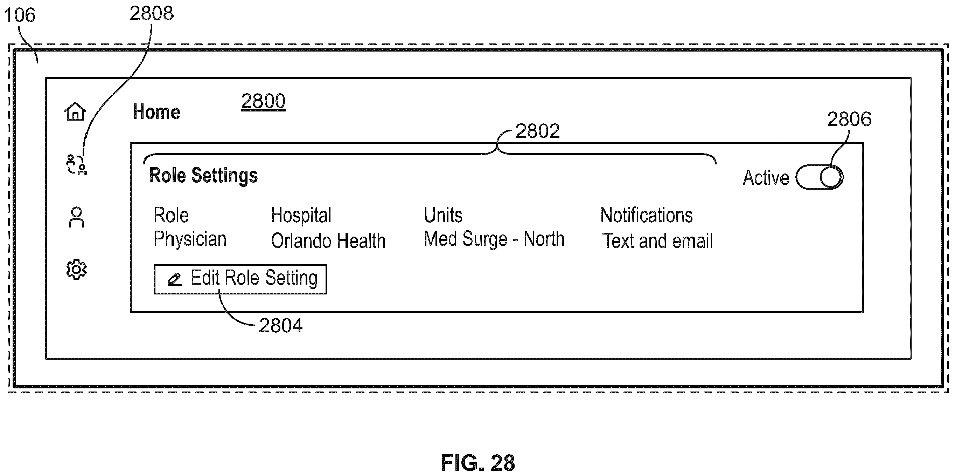

FIG. 27 illustrates an example of a method 2700 of providing a consultation to a caregiver 12 using the virtual care management application 110 installed or accessed on a primary device 106 or secondary device 108 of the remote care provider 16. The method 2700 is from the perspective of the remote care provider 16. The method 2700 includes an operation 2702 of signing into the virtual care management application 110, an operation 2704 of receiving a virtual care request from the caregiver 12, an operation 2706 of accepting the virtual care request, and an operation 2708 of joining a virtual patient room. Operations 2702-2708 will be described in more detail with respect to the various screens shown in FIGS. 28-39.

With respect to operation 2702, the remote care provider 16 can sign into the virtual care management application 110 using similar procedures as the caregiver 12 described above with respect to FIGS. 3-6. For example, the remote care provider 16 to sign into the virtual care management application 110 using single sign-on (SSO) credentials or by scanning their badge.

FIG. 28 illustrates an example of a home screen 2800 that is generated on the primary device 106 of the remote care provider 16 after the remote care provider 16 has successfully signed into the virtual care management application 110. As described above, the primary device 106 of the remote care provider 16 can be a laptop, a tablet computer, or a desktop computer.

The home screen 2800 includes one or more role settings 2802 such as the role or position of the remote care provider, the hospital or clinic where the remote care provider 16 is admitted, the units within the hospital or clinic to which the remote care provider is assigned, and the types of notifications that the remote care provider can receive on their primary and secondary devices 106, 108. The remote care provider 16 can select an edit role settings icon 2804 to edit any one of the role settings 2802. For example, the remote care provider 16 can select the edit role settings icon 2804 to receive only certain types of virtual care requests such as based on the request type, healthcare network, or location or type of clinical care environment.

For example, after the remote care provider 16 signs into the virtual care management application 110, the remote care provider 16 can select the type of virtual care request notifications they will receive. The remote care provider 16 may also filter the virtual care requests that they receive by request type or by location (e.g., facility or unit) of the clinical care environment 10. For example, the remote care provider 16 can select the request types that they wish to receive notifications for such as stroke, and other request types and medical symptoms.

The remote care provider can select or unselect an activation switch 2806 to indicate whether the remote care provider 16 is active (e.g., on duty) or unactive (e.g., on break, off duty, etc.). When the activation switch 2806 is selected to indicate that the remote care provider 16 is active, the remote care provider receives virtual care requests on their primary and secondary devices 106, 108. When the activation switch 2806 is selected to indicate that the remote care provider 16 is unactive, the remote care provider 16 does not receive the virtual care requests.

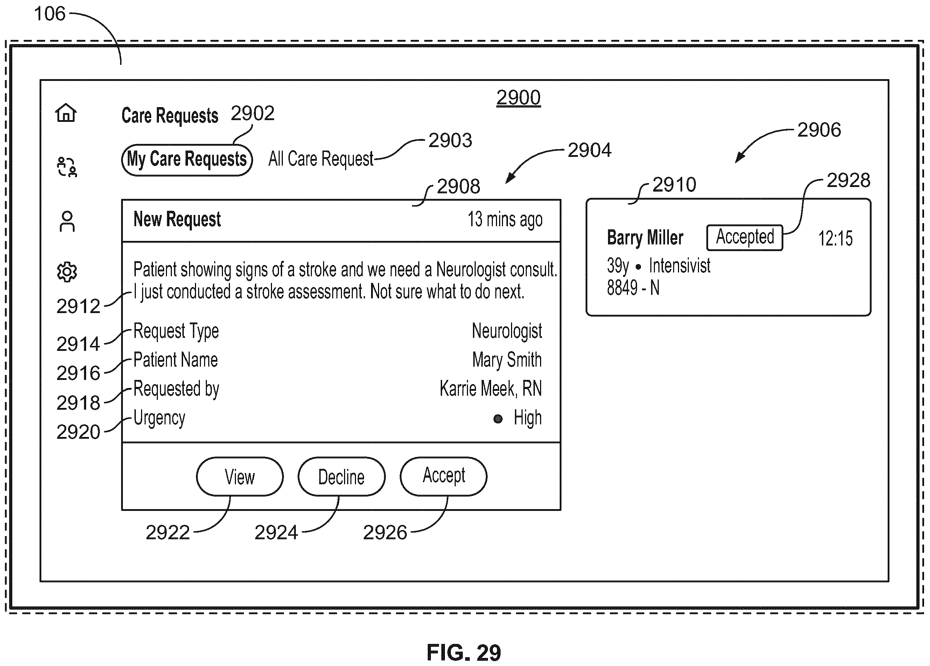

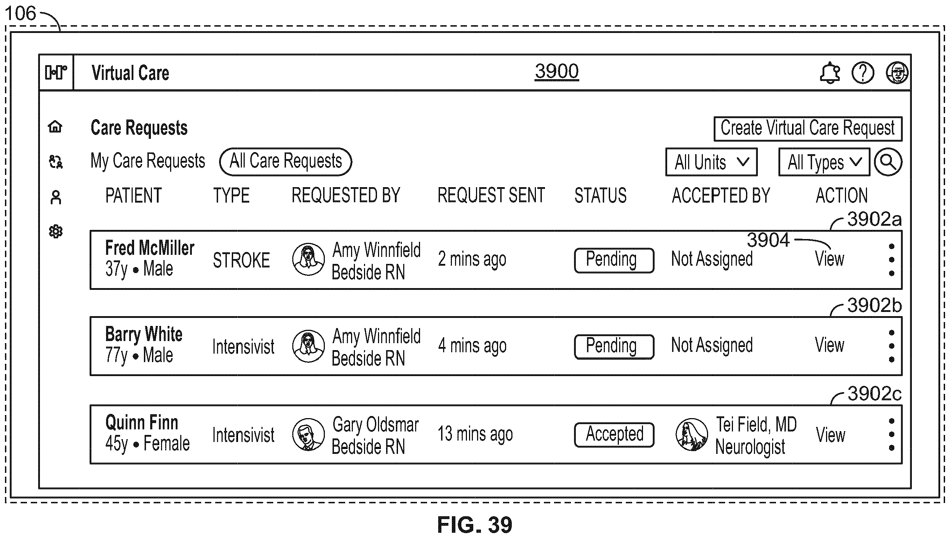

FIG. 29 illustrates an example of a care requests screen 2900 that is generated on the primary device 106 of the remote care provider 16 in response to the remote care provider selecting a view care requests icon 2808 on the home screen 2800 of FIG. 28. As shown in FIG. 29, a my care requests tab 2902 includes a first column 2904 of new virtual care requests and a second column 2906 of accepted virtual care requests. In the example shown, a new virtual care request 2908 is displayed under the first column 2904, and an accepted virtual care request 2910 is displayed under the second column 2906. The virtual care requests can be received from the clinical care environment 10 shown in FIG. 1, and also from other medical facilities that are geographically dispersed. Accordingly, the care requests screen 2900 can display virtual care requests for patients across multiple facilities in one, single view. In addition to the my care requests tab 2902, the care requests screen 2900 can also include an all care requests tab 2903 which will be described in more detail with reference to FIG. 39.

The new virtual care request 2908 includes information added by the caregiver 12 in accordance with the steps shown in FIG. 8, and the screens shown in FIGS. 9-14. For example, the new virtual care request 2908 can include a request summary 2912 (e.g., "Patient showing signs of a stroke. I just conducted a stroke assessment. Not sure what to do next."), a request type 2914 (e.g., "Neurologist"), the patient's name 2916 (e.g., "Mary Smith"), the name of the person who submitted the virtual care request 2918 (e.g., "Karrie Meek, RN"), and an urgency level 2920 (e.g., "High", "Moderate", or "Low"). In some examples, the virtual care requests may not include all of this information, or may include additional information. The urgency level 2920 can be helpful for the remote care provider 16 to prioritize the new virtual care requests.

The new virtual care request 2908 includes options for the remote care provider 16 to respond. For example, the remote care provider 16 can select a view icon 2922 to view additional information related to the patient 14 such as information from an electronic medical record (EMR) of the patient 14, vital signs, lab results, and the like. The remote care provider can select a decline icon 2924 to decline the new virtual care request 2908 or the remote care provider can select an accept icon 2926 to accept the new virtual care request 2908.

In the second column 2906, a list of accepted virtual care requests 2910 can be displayed. Each accepted virtual care request 2910 can display information such as the name of the patient (e.g., Barry Miller"), the age of the patient (e.g., "39 years old"), the request type (e.g., Intensivist") and the patient's medical record number (e.g., "8849-N"). Additionally, each accepted virtual care request 2910 can include a status box 2928 that indicates that the virtual is pending, and may include a time stamp such as when the care request was received or accepted.

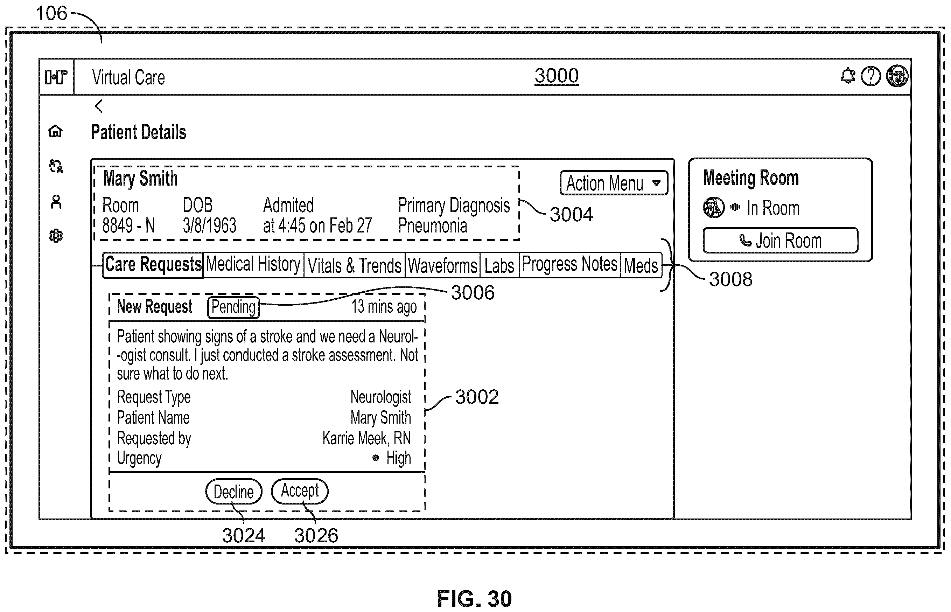

FIG. 30 illustrates an example of a patient details screen 3000 that can be generated on the primary device 106 of the remote care provider 16 in response to selecting the view icon 2922 in the new virtual care request 2908 displayed on the care requests screen 2900 of FIG. 29. The patient details screen 3000 includes additional information on the condition of the patient 14 that provide context to help the remote care provider 16 determine whether to accept or decline a virtual care request 3002 for the patient 14, and/or to help the guide the consultation from the remote care provider once the virtual care request 3002 is accepted. The information on the condition of the patient can be acquired from the electronic medical record (EMR) of the patient, or from other devices monitoring the status of the patient 14 such as a vital signs monitoring device or a hospital bed, which can provide the information through a digital health gateway.

In the example shown in FIG. 30, the virtual care request 3002 includes a status box 3006 that indicates that the virtual care request 3002 is pending. Based on the information of the patient 14 provided in the patient details screen 3000, the remote care provider 16 can select a decline icon 3024 to decline the virtual care request 3002, or can select an accept icon 3026 to accept the virtual care request 3002. In the patient details screen 3000, the virtual care request 3002 includes similar information as the new virtual care request 2908 shown in FIG. 29.

The patient details screen 3000 includes a primary information area 3004 that can provide basic information on the patient 14 such as the patient's name, room number, date of birth, date and time of admission, and primary diagnosis. The patient details screen 3000 further includes a plurality of tabs 3008 that can be selected by the remote care provider 16 to view additional information on the patient 14. In the illustrative example shown in FIG. 30, the plurality of tabs 3008 includes, without limitation, a care requests tab to view current and previous care requests that were submitted for the patient 14, a medical history tab that provides the medical history of the patient 14, a vitals and trends tab to view current vital signs and trends of the patient 14, a waveforms tab to view waveforms of the patient 14, a lab results tab to view lab results of the patient 14, a progress notes tab to view notes on the progress of the patient 14, and a medications tab to view a list of the medications that the patient 14 is taking.

The plurality of tabs 3008 and the information displayed on the patient details screen 3000 may be different for different request types. For example, a patient details screen for an intensivist request type may display different tabs and information than a patient details screen for a psychologist request type. In some examples, the tabs and information displayed on the patient detail screens for the different request types can be configured by an administrator during setup of the virtual care management application 110. Accordingly, the tabs and information shown in the patient details screen 3000 of FIG. 30 are provided by way of illustrative example, and it is possible for the patient details screen 3000 to omit some of these tabs, or to include additional tabs for viewing additional types of patient information.

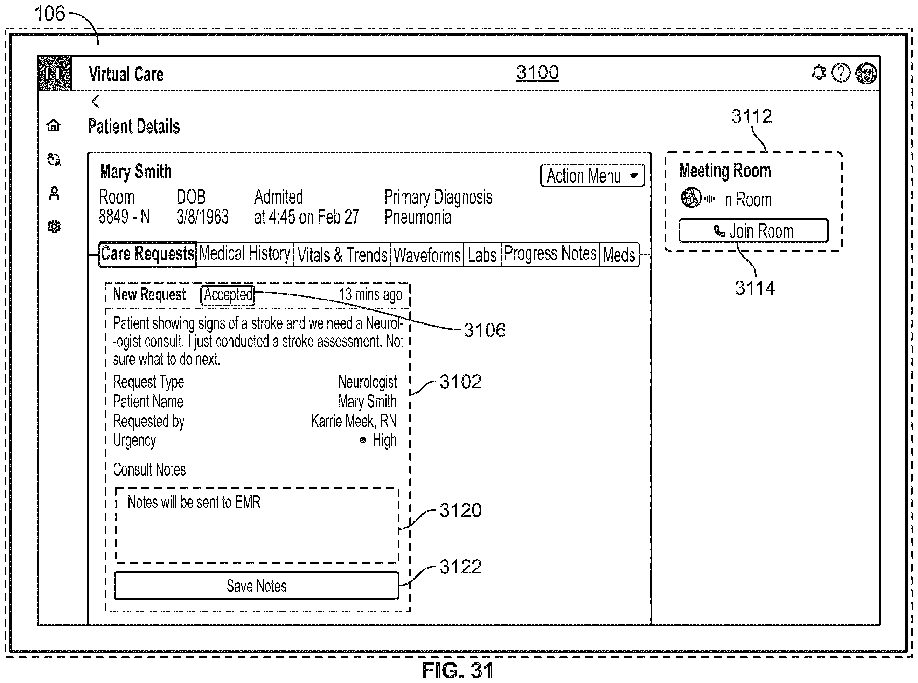

FIG. 31 illustrates an example of a patient details screen 3100 that can be generated on the primary device 106 of the remote care provider 16 in response to the remote care provider selecting the accept icon 3026 to accept the virtual care request 3002 of the patient details screen 3000 shown in FIG. 30. As shown in FIG. 31, a care request 3102 includes a status box 3106 that displays that the care request 3102 has been accepted. Additionally, the care request 3102 includes a consultation notes box 3120 that the remote care provider 16 can use to type notes before, during, or after the consultation with the caregiver 12 and patient 14. A save icon 3122 allows the remote care provider 16 to save the notes to the EMR of the patient 14.

The patient details screen 3100 further includes a meeting room module 3112 that indicates whether the caregiver 12 who submitted the care request 3102 has joined a virtual patient room (e.g., after the caregiver 12 receives a notification that the remote care provider 16 accepted the care request 3102), and a join meeting room icon 3114 that the remote care provider 16 can select to enter the virtual patient room with the caregiver 12. In this example, the meeting room module 3112 indicates that the caregiver 12 has joined the virtual patient room.

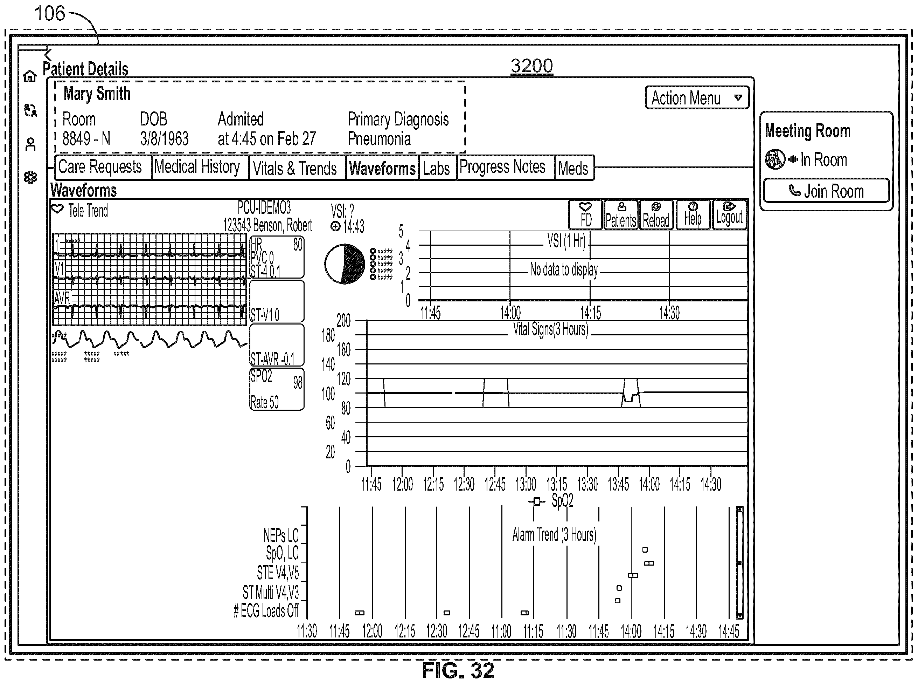

FIG. 32 illustrates another example of a patient details screen 3200 that is generated on the primary device 106 of the remote care provider 16. In this example, the patient details screen 3200 displays information in response to a selection of the waveforms tab. In some examples, the virtual care management application 110 allows the remote care provider 16 to configure the way data is displayed on the patient details screens, and to set data display defaults to enable more efficient use the screens and faster diagnosis.

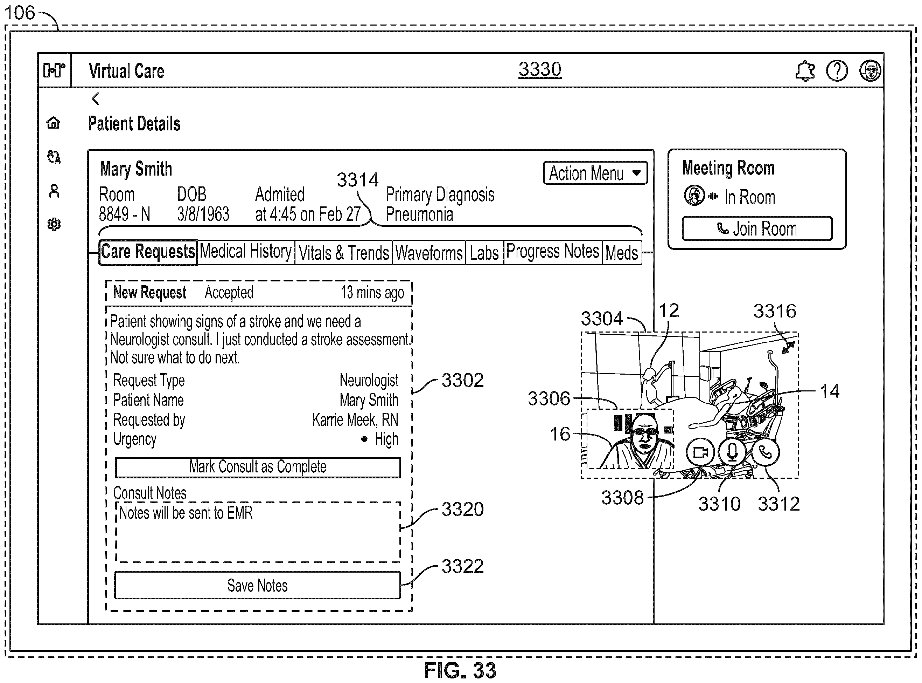

FIG. 33 illustrates an example of a patient details screen 3300 that can be generated on the primary device 106 of the remote care provider 16 in response to the remote care provider 16 selecting the join meeting room icon 3114 from the patient details screen 3100 of FIG. 31. The patient details screen 3300 includes similar items as the patient details screen 3100 shown in FIG. 31 such as a care request 3302 that includes a consultation notes box 3320 that the remote care provider 16 can use to type notes, and a save icon 3322 allows the remote care provider 16 to save the notes to the EMR of the patient 14. Also, the patient details screen 3300 includes a plurality of tabs 3314 that can be selected by the remote care provider 16 before, during, or after the consultation with the caregiver 12 and patient 14. The plurality of tabs 3314 are substantially similar or the same as the plurality of tabs shown in the patient details screen 3000 of FIG. 30.

As shown in FIG. 33, the patient details screen 3300 includes a window 3304 that displays a virtual patient room that allows a consultation between the caregiver 12 and the remote care provider 16. The window 3304 can include a live video feed of the caregiver 12 who submitted the care request 3302 and in some instances the patient 14. The remote care provider 16 can communicate with the caregiver 12 and patient 14 using speakers and a microphone of the primary device 106, while viewing the caregiver 12 and patient 14 in the window 3304.

In some examples, multiple cameras are provided in the patient's room, and the virtual care management application 110 allows the remote care provider 16 to control the cameras to adjust the live video feed inside the window 3304. For example, the remote care provider 16 can select certain cameras for viewing certain angles of the patient 14, or can select all of the cameras to have a 360 degree view of the patient 14 and their room.

A window 3306 can also be provided within the window 3304 to display a live video feed of the remote care provider 16 acquired from the camera of the primary device 106. Thus, in some examples, the patient details screen 3300 can provide a two-way video conference between the remote care provider 16 and the caregiver 12 and patient 14.

Additionally, the virtual patient room can be a central location where multiple communication applications are integrated to incorporate additional users of the virtual care management application 110 in the conference between the caregiver 12, patient, 14, and remote care provider 16. For example, additional remote care providers, additional caregivers, hospitalists, case managers, and family members can all participate in collaborative calls to share information during rounding, diagnosis, or discharge. Thus, the virtual care management application 110 can connect multiple users and related applications in a virtual patient room for each patient. Also, the collaborative virtual patient rooms can be used in home care scenarios.

The window 3304 can include a video camera icon 3308 that the remote care provider 16 can select to turn on and off the camera of the primary device 106, and thereby allow or block the live video feed of the remote care provider inside the window 3206. The window 3304 can also include a microphone icon 3310 that the remote care provider 16 can select to turn off and on the microphone of the primary device 106, and thereby mute and unmute the remote care provider 16. The window 3304 can also include a hang up icon 3312 that the remote care provider 16 can select to terminate the video conference with the caregiver 12 and patient 14. The window 3304 can further include an expansion arrow 3316 that the remote care provider 16 can select to expand the window 3304 to a full screen view.

In some examples, the virtual patient rooms can be recorded and stored to provide enhanced feedback for patient care. For example, the virtual care management application 110 can perform natural language processing (NLP) to detect who is talking, transcribe the conversation, and pull out important medical terms that were mentioned. This can help improve shift handoff and discharge instructions by reducing the exchange of misinformation.

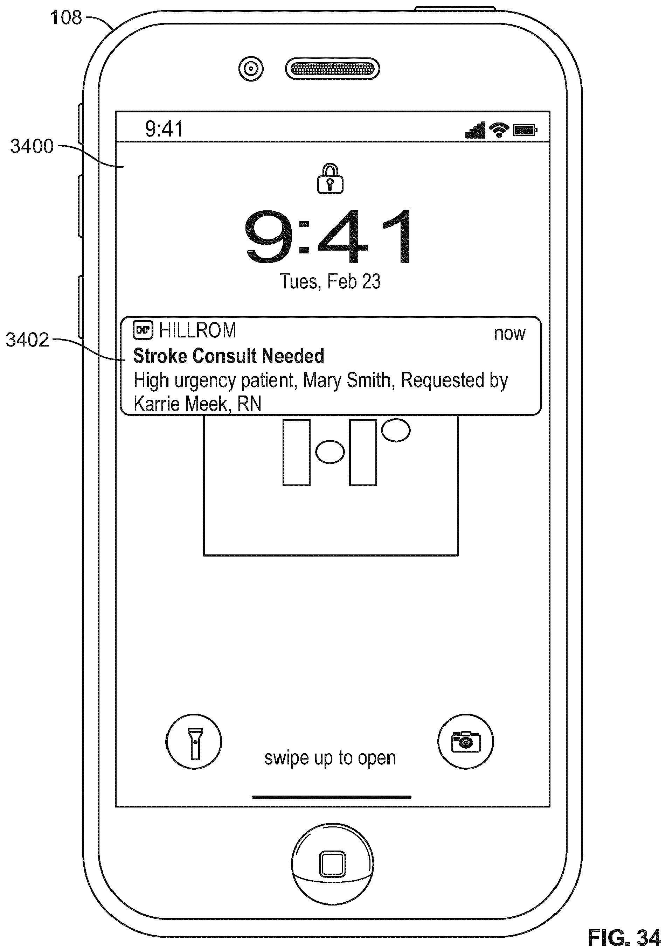

FIG. 34 illustrates an example of a notification 3402 displayed on a secondary device 108 of the remote care provider 16 by the virtual care management application 110. In this example, the secondary device 108 of the remote care provider 16 is a smartphone. The notification 3402 is displayed on a lock screen 3400 of the secondary device 108. The notification 3402 includes a message regarding a submitted care request such as "Stroke Consult Needed. High urgency patient, Mary Smith, requested by Karrie Meek, RN". As another example, the notification 3402 can indicate whether the caregiver 12 who submitted an accepted care request has joined a virtual patient room. Upon receiving the notification 3402, the remote care provider 16 can open the virtual care management application 110 on the secondary device 108 to view, accept or decline, and join the virtual patient room.

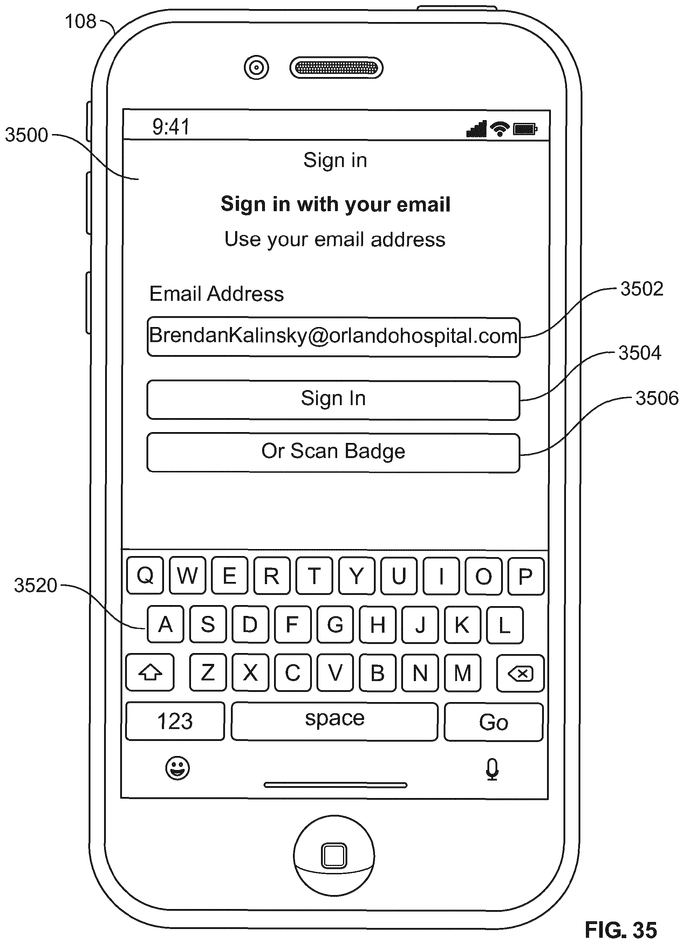

FIG. 35 illustrates an example of a sign in screen 3500 generated on the secondary device 108 of the remote care provider 16 by the virtual care management application 110. The sign in screen 3500 can be generated on the secondary device 108 in response to the remote care provider 16 receiving the notification 3402 on the lock screen 3400 of FIG. 34, and opening up the virtual care management application 110 on the secondary device 108. The sign in screen 3500 is similar to sign in screens described above for the primary device 102 of the caregiver 12. For example, the sign in screen 3500 provides a keyboard 3520 that the remote care provider 16 can use to type their email address or username into a text field 3502, and a sign in option 3504 that can be selected to sign into the virtual care management application 110. Alternatively, the remote care provider 16 can select a scan badge option 3506 to sign into the virtual care management application 110 by scanning their badge, similar to how the caregiver 12 can scan their badge to sign into the virtual care management application 110, as shown in FIG. 6.

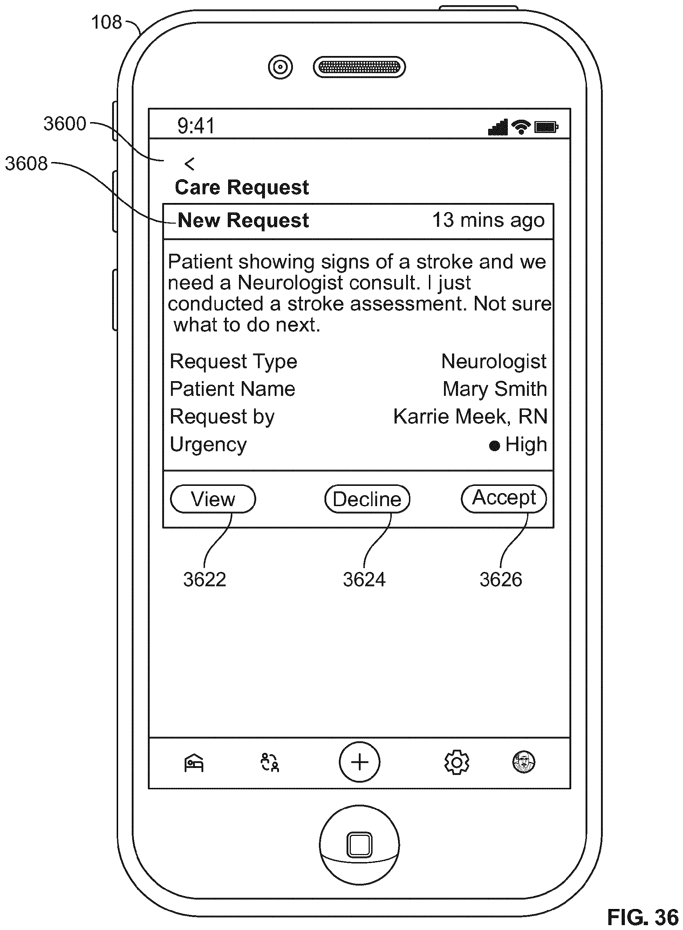

FIG. 36 illustrates an example of a care requests screen 3600 that is generated on the secondary device 108 of the remote care provider 16. The care requests screen 3600 includes a new care request 3608 that is similar to the one shown in the care requests screen 2900 displayed on the primary device 106 of the remote care provider 16, as shown in FIG. 29. For example, the new care request 3608 can include information that was added to the care request by the caregiver 12 in accordance with the steps shown in FIG. 8, and the screens shown in FIGS. 9-14. For example, the new care request 3608 can include a summary (e.g., "Patient showing signs of a stroke. I just conducted a stroke assessment. Not sure what to do next."), a request type (e.g., "Neurologist"), the patient's name (e.g., "Mary Smith"), the name of the person who submitted the care request (e.g., "Karrie Meek, RN"), and an urgency level (e.g., "High", "Moderate", or "Low"). These details are provided by way of example, and the new care request 3608 may not include all of this information, or may include additional information.

The new care request 3608 further includes options for the remote care provider 16 to submit a response. For example, the new care request 3608 can include a view icon 3622 that can be selected to view additional information related to the patient 14 such as information from the patient's electronic medical record (EMR) including the information shown in the patient details screens shown in FIGS. 30-33. The new care request 3608 can also include a decline icon 3624 that the remote care provider can select to decline the new care request 3608 and an accept icon 3626 that the remote care provider can select to accept the new care request 3608.

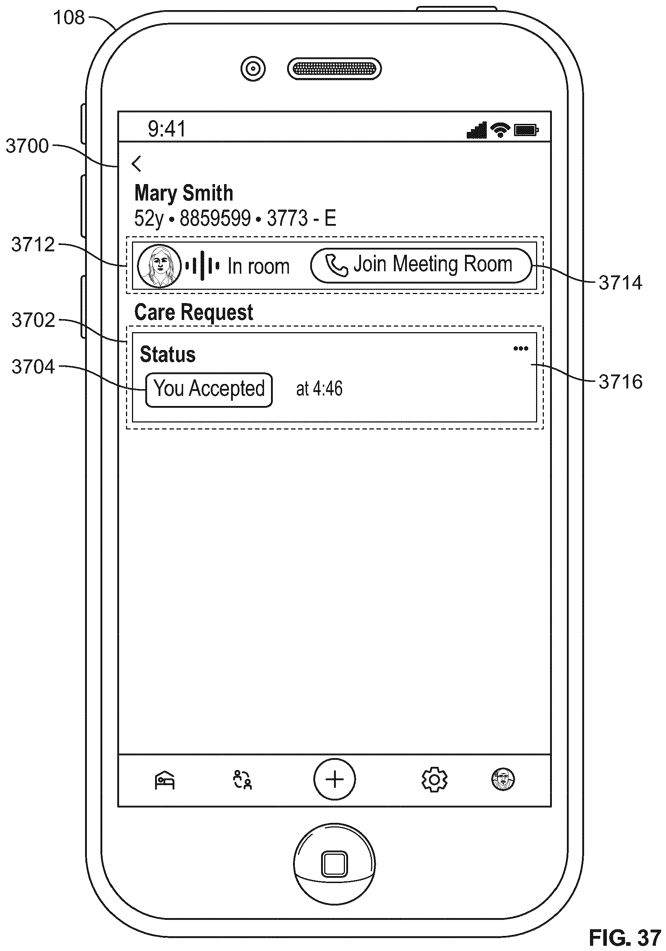

FIG. 37 illustrates an example of an accepted care request screen 3700 that is generated on the secondary device 108 of the remote care provider 16 in response to the remote care provider 16 selecting the accept icon 3626 on the new care request 3608 shown in FIG. 36. The accepted care request screen 3700 displays a care request 3702 that includes a status box 3704 displaying a status of the care request. In this example, the status box 3704 indicates that the care request 3702 was accepted. In some examples, the care request 3702 can include a time stamp next to the status box 3704 to indicate the time the care request was accepted or received. The care request 3702 can further include an edit icon 3716 that the remote care provider 16 can select to edit their response to the care request 3702 such as to decline the care request 3702.

The accepted care request screen 3700 further displays a meeting room module 3712 that indicates whether the caregiver 12 who submitted the care request 3702 has joined a virtual patient room (i.e., in response to the remote care provider 16 accepting the care request 3702), and a join meeting room icon 3714 that the remote care provider 16 can select to enter the virtual patient room for a consultation with the caregiver 12. In the example shown in FIG. 37, the meeting room module 3712 indicates that the caregiver 12 has joined the virtual patient room.

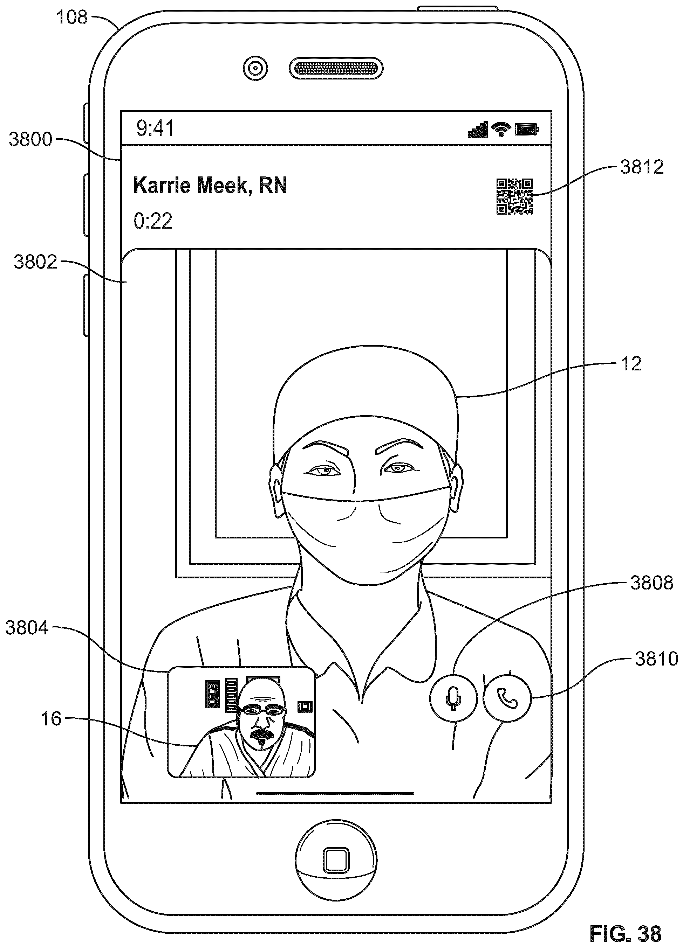

FIG. 38 illustrates an example of a meeting room screen 3800 that is generated on the secondary device 108 of the remote care provider 16 in response to the remote care provider 16 selecting the join meeting room icon 3714 from the meeting room module 3712 shown in FIG. 37. The meeting room screen 3800 is similar to the meeting room screen 2200 shown in FIG. 22 that is generated on the primary device 102 of the caregiver 12 by the virtual care management application 110. For example, the meeting room screen 3800 can display the name of the caregiver 12 who submitted the care request and can also display a duration of the meeting. The meeting room screen 3800 further includes a window 3802 that displays a live video feed of the caregiver 12. The remote care provider 16 can communicate with the caregiver 12 using the audio system of the secondary device 108, while viewing the caregiver 12 in the window 3802.

The meeting room screen 3800 can further include a window 3804 that displays a live video feed of the remote care provider 16 acquired from the camera of the secondary device 108. Thus, the meeting room screen 3800 can provide a two-way video conference between the remote care provider 16 and caregiver 12. The meeting room screen 3800 can include a video camera icon that the remote care provider 16 can select to turn on and off the camera of the secondary device 108. The meeting room screen 3800 can also include a microphone icon 3808 that the remote care provider 16 can select to turn off and on the microphone of the secondary device 108 to mute and unmute the remote care provider 16, and a hang up icon 3810 that the remote care provider 16 can select to terminate the consultation with the caregiver 12.

In some examples, the meeting room screen 3800 may also display a view of patient data on the secondary device 108, such as the data that is shown in FIGS. 32 and 33 on the primary device 106. The patient data can be formatted to fit on the meeting room screen 3800 displayed on the secondary device 108 such as to fit on a smartphone display.

In some examples, the meeting room screen 3800 can further include a link 3812 that the remote care provider can select to transfer the consultation with the caregiver 12 to another device such as the primary device 106 of the remote care provider. The virtual care management application 110 can allow the remote care provider 16 to transfer the consultation from the secondary device 108 to the primary device 106 using similar procedures that allow the caregiver 12 to transfer a virtual patient room between the primary and secondary devices 102, 104.

FIG. 39 illustrates an example of an all care requests screen 3900 that is generated on the primary device 106 of the remote care provider 16 in response to the remote care provider selecting the all care requests tab 2903 included in the care requests screen 2900 of FIG. 29. The all care requests screen 3900 includes a list of care requests 3902a-3902d submitted regardless of whether the care request is pending or accepted, the caregiver 12 who submitted the care request, or the remote care provider who accepted the care request. The all care requests screen 3900 can provide the ability to search, sort, and filter through the list of care requests 3902a-3902d.

Each care request 3902 includes information such as a patient name, a type of care request, a person who submitted the care request, a time stamp of when the care request was submitted, a status of the care request, a name of the remote care provider who accepted the care request, and the like. Additionally, each care request 3902 can include a link 3904 that can be selected to view additional information related to the care request.

As an illustrative example, a care request 3902a is for a patient Fred McMiller for a stroke consultation, was submitted by Amy Winnfield (a bedside RN) about 2 minutes ago, and is currently pending such that it has not yet been accepted by a remote care provider. As another illustrative example, a care request 3902c is for a patient Quinn Finn for an intensivist consultation, was submitted by Gary Oldsmar (a bedside RN) about 13 minutes ago, and was accepted by a remote care provider named Tei Field MD who is a neurologist.

In view of the foregoing, the graphical user interfaces and screens that are shown in the figures and described above provide a technical effect by improving efficiencies related to the operation of the devices 102, 104, 106, 108 as a means to send and receive virtual care requests for remote care management such as between a rural, community hospital and one or more remote care providers located in a different city, county, or state. These efficiencies can extend to how data is displayed and interactions with the data on the devices 102, 104, 106, 108. Moreover, these graphical user interfaces and screens integrate the remote care management into a practical application that is more effective and efficient than previous methods.

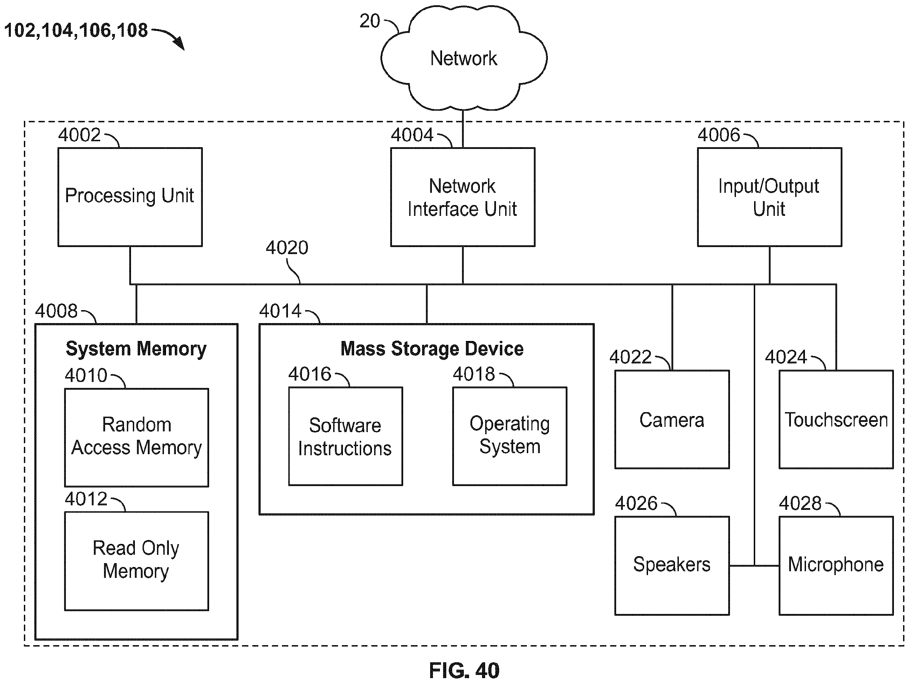

FIG. 40 schematically illustrates in more detail an example of a device 102, 104, 106, 108 that can be used by the caregiver 12 and remote care providers 16 to implement aspects of the virtual care management application 110. The device 102, 104, 106, 108 includes a processing unit 4002, a system memory 4008, and a system bus 4020 that couples the system memory 4008 to the processing unit 4002. The processing unit 4002 is an example of a processing device such as a central processing unit (CPU). The system memory 4008 includes a random-access memory ("RAM") 4010 and a read-only memory ("ROM") 4012. A basic input/output logic having basic routines that help to transfer information between elements within the device 102, 104, 106, 108, such as during startup, is stored in the ROM 4012.

The device 102, 104, 106, 108 can also include a mass storage device 4014 that is able to store software instructions and data. The mass storage device 4014 is connected to the processing unit 4002 through a mass storage controller (not shown) connected to the system bus 4020. The mass storage device 4014 and its associated computer-readable data storage media provide non-volatile, non-transitory storage for the device 102, 104, 106, 108.

Although the description of computer-readable data storage media contained herein refers to a mass storage device, it should be appreciated by those skilled in the art that computer-readable data storage media can be any available non-transitory, physical device or article of manufacture from which the device can read data and/or instructions. In certain embodiments, the computer-readable storage media comprises entirely non-transitory media. The mass storage device 4014 is an example of a computer-readable storage device.

Computer-readable data storage media include volatile and non-volatile, removable and non-removable media implemented in any method or technology for storage of information such as computer-readable software instructions, data structures, program modules or other data. Example types of computer-readable data storage media include, but are not limited to, RAM, ROM, EPROM, EEPROM, flash memory or other solid-state memory technology, or any other medium which can be used to store information, and which can be accessed by the device.

The device 102, 104, 106, 108 operates in a networked environment using logical connections to devices through the communications network 20. The device 102, 104, 106, 108 connects to the communications network 20 through a network interface unit 4004 connected to the system bus 4020. The network interface unit 4004 can also connect to additional types of communications networks and devices, including through Bluetooth, Wi-Fi, and cellular.

The network interface unit 4004 may also connect the device 102, 104, 106, 108 to additional networks, systems, and devices such as a digital health gateway, electronic medical record (EMR) system, vital signs monitoring devices, and clinical resource centers.

The device 102, 104, 106, 108 can also include an input/output unit 4006 for receiving and processing inputs and outputs from a number of peripheral devices. Examples of peripheral devices may include, without limitation, a camera 4022, a touchscreen 4024, speakers 4026, a microphone 4028, and similar devices used for voice and video communications.

The mass storage device 4014 and the RAM 4010 can store software instructions and data. The software instructions can include an operating system 4018 suitable for controlling the operation of the device 102, 104, 106, 108. The mass storage device 4014 and/or the RAM 4010 also store software instructions 4016, that when executed by the processing unit 4002, cause the device to provide the functionality of the device 102, 104, 106, 108 discussed herein.

The various embodiments described above are provided by way of illustration only and should not be construed to be limiting in any way. Various modifications can be made to the embodiments described above without departing from the true spirit and scope of the disclosure.

## Claims

1. A device for remote care management, the device comprising:
at least one processor; and
a memory storing instructions which, when executed by the at least one processor, cause the device to:
provide a screen configurable by a caregiver to create a virtual care request;
receive a selection on the screen to submit the virtual care request;
receive an acceptance of the virtual care request from a remote care provider; and
provide a connection for the caregiver to enter a virtual patient room with the remote care provider.

2. The device of claim 1, wherein the screen allows the caregiver to create the virtual care request by identifying a patient, entering a request type, and entering a request summary.

3. The device of claim 2, further comprising:
a camera; and
wherein the screen allows the caregiver to identify the patient by scanning a label with the camera, the label including machine-readable data that identifies the patient.

4. The device of either claim 2 or claim 3, wherein the identity of the patient is confirmed using real-time locating system data.

5. The device of any preceding claim, wherein the connection allows the caregiver to transfer the virtual patient room to a second device by providing machine readable data or a code recognizable by the second device for transferring the virtual patient room to the second device.

6. The device of any preceding claim, wherein the memory stores further instructions which, when executed by the at least one processor, cause the device to:
provide a notification that the remote care provider entered the virtual patient room.

7. A method of remote care management, the method comprising:
providing a screen configurable by a caregiver to create a virtual care request;
receiving a selection to submit the virtual care request;
receiving an acceptance of the virtual care request from a remote care provider; and
providing a connection for the caregiver to enter a virtual patient room with the remote care provider.

8. The method of claim 7, further comprising:
allowing the caregiver to create the virtual care request by identifying a patient, entering a request type, and entering a request summary.

9. The method of claim 8, further comprising:
allowing the caregiver to identify the patient by scanning a label that includes machine-readable data identifying the patient.

10. The method of any one of claims 7 to 9, further comprising:
providing machine readable data or a code recognizable by a second device for transferring the virtual patient room to the second device.

11. The method of any one of claims 7 to 10, further comprising:
providing a notification that the remote care provider entered the virtual patient room.

12. A device for remote care management, the device comprising:
at least one processor; and
a memory storing instructions which, when executed by the at least one processor, cause the device to:
receive a virtual care request;
display information added by a caregiver to the virtual care request;
display controls to decline or accept the virtual care request; and
in response to receiving an acceptance of the virtual care request, provide a connection for a remote care provider to enter a virtual patient room with the caregiver.

13. The device of claim 12, wherein the virtual care request includes a patient identity, a request type, a request summary, and an urgency level.

14. The device of either claim 12 or claim 13, wherein the memory stores further instructions which, when executed by the at least one processor, cause the device to:
in response to the remote care provider entering the virtual patient room, providing a screen that includes a window displaying a live video feed of the caregiver.

15. The device of claim 14, wherein the screen further includes a consultation notes box allowing the remote care provider to type notes during a consultation with the caregiver in the virtual patient room, and to save the notes to an electronic medical record of a patient.
